# FASCICULE DE BREVET EUROPEEN

(11) **EP 2 510 119 B1**
(45) Date de publication et mention de la délivrance du brevet: **21.10.2020**
(21) Numéro de dépôt: 10803594.0
(22) Date de dépôt: 09.12.2010
(51) Int. Cl.: C12Q 1/68

(54) **UTILISATION DE LA DIVERSITE COMBINATOIRE DU REPERTOIRE DES LYMPHOCYTES T COMME MARQUEUR PRONOSTIQUE D'UN CANCER**
VERWENDUNG DER KOMBINATORISCHEN DIVERSTÄT DES T-LYMPHOZYTENREPERTOIRES ALS PROGNOSTISCHE KREBSMARKER
USE OF THE COMBINATORIAL DIVERSITY OF T-LYMPHOCYTE REPERTOIRE AS A PROGNOSTIC MARKER OF CANCER

(30) Priorité: 09.12.2009 EP 09290922
(43) Date de publication de la demande: 17.10.2012
(73) Titulaire: Adaptive Biotechnologies Corp., Seattle, Washington 98102 (US)
(72) Inventeur: CAUX, Christophe, F-01360 Bressolles (FR); MOURET, Jean-François, F-38500 Coublevie (FR); MENETRIER-CAUX, Christine, F-01360 Bressolles (FR); PASQUAL, Nicolas, F-38100 Grenoble (FR); BACHELOT, Thomas, F-69500 Bron (FR); MANUARII, Manuel, F-38600 Fontaine (FR); BLAY, Jean-Yves, F-38290 Frontonas (FR)
(74) Mandataire: Boult Wade Tennant LLP
(86) Numéro de dépôt international: PCT/FR2010/000829
(87) Numéro de publication internationale: WO 2011/070256

(56) Documents cités:
- WO-A2-2009/095567
- RAY-COQUARD I ET AL.: "Lymphopenia as a prognostic factor for overall survival in advanced carcinomas, sarcomas, and lymphomas", CANCER RESEARCH, vol. 69, no. 13, juillet 2009 (2009-07), pages 5383-5391, XP002581656, cité dans la demande
- BORG C ET AL.: "CD4 lymphopenia as a risk factor for debrile neutropenia and early death after cytotoxic chemotherapy in adult patients with cancer", CANCER, vol. 101, 2004, pages 2675-2680, XP002581657, cité dans la demande
- DONGEN VAN J J M ET AL: "DESIGN AND STANDARDIZATION OF PCR PRIMERS AND PROTOCOLS FOR DETECTION OF CLONAL IMMUNOGLOBULIN AND T-CELL RECEPTOR GENE RECOMBINATIONS IN SUSPECT LYMPHOPROLIFERATIONS: REPORT OF THE BIOMED-2 CONCERTED ACTION BMH4-CT98-3936", LEUKEMIA, MACMILLAN PRESS LTD, US LNKD- DOI:10.1038/SJ.LEU.2403202, vol. 17, 1 janvier 2003 (2003-01-01), pages 2257-2317, XP008062303, ISSN: 0887-6924
- FUSCHIOTTI ET AL: "Analysis of the TCR alpha-chain rearrangement profile in human T lymphocytes", MOLECULAR IMMUNOLOGY, PERGAMON, GB LNKD- DOI:10.1016/J.MOLIMM.2007.02.017, vol. 44, no. 13, 6 mai 2007 (2007-05-06), pages 3380-3388, XP022062327, ISSN: 0161-5890 cité dans la demande
- PASQUAL N ET AL: "Quantitative and qualitative changes in V-J alpha rearrangements during mouse thymocytes differentiation: implication for a limited T cell receptor alpha chain repertoire", 4 novembre 2002 (2002-11-04), THE JOURNAL OF EXPERIMENTAL MEDICINE, ROCKEFELLER UNIVERSITY PRESS, UNITED STATES LNKD- DOI:10.1084/JEM.20021074, PAGE(S) 1163 - 1173, XP002322207, ISSN: 0022-1007 le document en entier
- BAUM P D AND MCCUNE J M: "Direct measurement of T-cell receptor repertoire diversity with AmpliCot", NATURE METHODS, vol. 3, no. 11, novembre 2006 (2006-11), pages 895-901, XP002581658, cité dans la demande
- MARODON G ET AL.: "High diversity of the immune repertoire in humanized NOD.SCID.gammac-/- mice", EUROPEAN JOURNAL OF IMMUNOLOGY, vol. 39, juillet 2009 (2009-07), pages 2136-2145, XP002581659, cité dans la demande
- WEI LUO ET AL: "Dynamic monitoring the TCR CDR3 spectratypes in patients with metastatic CRC treated with a combination of bevacizumab, irinotecan, fluorouracil, and leucovorin", CANCER IMMUNOLOGY, IMMUNOTHERAPY, SPRINGER, BERLIN, DE, vol. 59, no. 2, 4 August 2009 (2009-08-04) , pages 247-256, XP019757710, ISSN: 1432-0851

## Description

La présente invention concerne le domaine du pronostic en oncologie. Plus précisément, la présente invention vise à identifier, parmi les malades atteints d'un cancer solide donné, ceux qui présentent un risque accru de décès précoce, afin notamment de pouvoir leur offrir un suivi médical plus approprié, l'accès à des innovations thérapeutiques et d'espérer ainsi augmenter leurs chances de guérison ou au moins leur durée et qualité de vie. Elle vise par ailleurs à identifier un groupe à risque (ne répondant vraisemblablement pas au traitement de référence) pour une pathologie tumorale donnée, afin d'évaluer l'efficacité de nouvelles stratégies thérapeutiques et d'augmenter le rapport bénéfice/risque de candidats médicaments, favorisant ainsi leur approbation clinique.

Les cancers solides désignent la multiplication anormale de cellules dans des tissus et/ou des organes "solides" comme le sein ou la prostate, par opposition à la leucémie, un cancer qui touche le sang et la moelle osseuse.

Parmi les cancers solides, le cancer du sein est un des plus fréquents. Il s'agit essentiellement d'un cancer de la femme (en France, presque 10% des femmes développent un cancer du sein au cours de leur vie). Il est rare chez l'homme (moins d'un cancer du sein sur 100) mais est plus grave, le diagnostic étant souvent plus tardif. C'est le cancer le plus fréquent chez la femme et la première cause de mortalité parmi les cancers gynécologiques des pays développés. Si une partie de ces cancers (10 à 15 %) ont une origine génétique héréditaire, 85 à 90 % des cas ont des origines mal comprises (forme dite sporadique ou non-héréditaire).

Le cancer du sein est une masse résultant de la multiplication de cellules malignes dans la glande mammaire. Cette masse, si elle n'est pas dépistée et traitée, peut grossir et donner des métastases.

Il existe un grand nombre de traitements disponibles pour le cancer du sein, suivant le stade d'évolution et les paramètres inhérents au patient. Chaque situation doit être individualisée et traitée de façon optimale. Pour le cancer du sein localisé, le traitement a un objectif curatif. Il repose sur les cinq armes thérapeutiques que sont la chirurgie, la chimiothérapie, la radiothérapie, l'hormonothérapie et, plus récemment, l'immunothérapie. La chirurgie est l'étape indispensable du traitement curatif du cancer du sein, les autres traitements visant généralement à réduire le risque de métastase ou de rechute. Ils sont donc indiqués si le risque est important et si le bénéfice supposé du traitement est suffisant, car tous ces traitements ont des effets secondaires. Le bénéfice attendu doit donc être mis en balance avec le risque de complication.

Pour le cancer du sein métastatique, il est rare de pouvoir proposer un traitement curatif. Mais les traitements modernes permettent souvent de prolonger la vie du patient de plusieurs années. Le traitement du cancer du sein métastatique repose d'abord sur la chimiothérapie et l'hormonothérapie. Un traitement chirurgical ou par radiothérapie des sites métastatiques peut être envisagé soit dans un but curatif lorsque tous les sites sont accessibles à un traitement (par exemple dans le cas de métastase hépatique ou vertébrale unique) soit dans un but palliatif (par exemple, irradiation d'une métastase osseuse douloureuse).

De nouvelles stratégies thérapeutiques, notamment basées sur l'utilisation d'anticorps monoclonaux ou sur la stimulation du système immunitaire, sont évaluées et développées à un rythme soutenu. Par exemple, le Trastuzumab (Herceptin ®) cible le récepteur Her2 (ou CerbB2), qui est un récepteur membranaire permettant d'activer une des voies de la prolifération cellulaire, sur-exprimé dans 25% des cancers mammaires, souvent de mauvais pronostic. L'Herceptin® a d'abord été utilisée en situation palliative. Dans ce contexte, l'Herceptin® a permis, en moyenne, de doubler le temps de survie de ces patientes. Ajoutée à la chimiothérapie adjuvante, l'Herceptin ® en perfusion tous les 21 jours, pendant 12 mois, réduit de moitié le risque de rechute chez les patientes Her2+ et d'environ un tiers la mortalité.

Malgré ces avancées, la mortalité du cancer du sein reste importante, surtout lorsqu'il atteint un stade métastatique. Il existe toutefois une grande variabilité individuelle, avec une durée de survie allant de moins d'un mois à plusieurs années.

Plusieurs marqueurs de pronostic ont été étudiés pour déterminer si un patient atteint d'un cancer solide donné a un risque accru de décès précoce. A ce titre, on peut citer le taux d'hémoglobine, la présence de métastases hépatiques, le PS (*Performance Status* : quantification de l'état général du patient ; le PS est donc un chiffre représentatif de cet état général) ou le taux de polynucléaires neutrophiles (PNN) (Ray-Coquard et al., 2009) et la lymphopénie, particulièrement la lymphopénie T CD4+ identifiée par les inventeurs de la présente invention (Borg et al., 2004).

La présente invention propose un nouveau marqueur pronostic de décès précoce pour les patients atteints de cancer solide, plus puissant que certains marqueurs déjà décrits, et indépendant de ces derniers. Les inventeurs ont en effet mis en évidence qu'une diminution de la diversité immunitaire des lymphocytes T dans le sang est corrélée à un risque accru de décès précoce.

Chaque lymphocyte T fonctionnel possède un récepteur (TCR) qui reconnaît de façon spécifique un nombre limité de peptides antigéniques différents. De ce fait, un vaste répertoire de récepteurs est requis pour assurer la défense de l'individu contre les multiples infections, les proliférations malignes ou autres agressions qu'il est susceptible de rencontrer dans son environnement. Pour cela, le système immunitaire a développé un mécanisme d'assemblage d'un grand nombre de segments de gènes V, D, J positionnés de façon discontinue dans le génome. Ce mécanisme d'assemblage, appelé "recombinaison V(D)J", est indépendant d'une cellule à l'autre et permet d'obtenir un seul "fragment" de gène codant pour le TCR. Ce système permet, avec un nombre modeste de gènes, de générer un grand nombre de récepteurs différents. Chaque cellule utilise une combinaison de segments géniques selon des règles précises et obtient une chaîne TCR potentiellement unique.

Le principe de la recombinaison est basé sur la reconnaissance de séquences RSS spécifiques des gènes V(D)J et l'excision de la région chromosomique intercalée entre les deux gènes réarrangés. Chaque gène V et J possède, à une de ses extrémités, une séquence signal de recombinaison (RSS). Quant aux gènes D, ils en possèdent aux deux extrémités. Les RSS sont des séquences reconnues par les enzymes spécifiques de la recombinase, RAG I et RAG II, exprimées spécifiquement dans les lymphocytes. Ces protéines sont les actrices principales du réarrangement. Une fois associées aux protéines HMG (*High mobility group*), les enzymes RAG reconnaissent le nonamère du RSS grâce à leur homéodomaine et induisent une coupure entre le segment de gène V, D, J et l'heptamère, de façon à générer une extrémité codante et une extrémité signal. L'achèvement d'un réarrangement est obtenu après ligation des deux extrémités codantes V et J. Cette étape est précédée par l'action de l'enzyme TdT et d'une nucléase au niveau de la jonction V-J. Une fois réarrangé, le gène néoformé est transcrit puis épissé en ARNm avant d'être traduit en protéine membranaire.

Quatre mécanismes majeurs contribuent à générer la diversité du répertoire : 1) une diversité combinatoire qui correspond à la première étape de réarrangement entre un segment V et un segment J, éventuellement séparés par un segment D ; 2) une diversité jonctionnelle, générée au niveau de la jonction entre les segments de gènes réarrangés ; 3) des hypermutations somatiques dans les gènes réarrangés V-J et V-D-J ; 4) une diversité d'appariement des hétérodimères protéiques TCRα x TCRβ ou TCRγ x TCRδ.

La première étape de génération de diversité, appelée ici "diversité combinatoire", est basée sur le principe du réarrangement des gènes V(D)J. Le calcul de cette diversité consiste à estimer le nombre de combinaisons mV x nD x pJ possibles. Cette première étape de génération de diversité définit l'ordre de grandeur du répertoire. En effet, même si cette étape ne génère qu'une modeste variabilité de combinaison (de l'ordre de quelques milliers de combinaisons possibles par rapport au répertoire maximal théorique estimé à 10¹⁵ (Davis and Bjorkman, 1988), la diversité combinatoire maximale est directement liée au nombre de gènes V, D et J initialement disponibles : les deux autres étapes de génération de la diversité amplifient de manière exponentielle la diversité du répertoire primaire.

La diversité de jonction permet de générer une très grande variabilité au niveau de la région CDR3 du récepteur en contact avec le peptide antigénique. Deux mécanismes contribuent à augmenter la diversité jonctionnelle : 1) le premier mécanisme est dû à l'ajout de nucléotides P (pour palindromiques), provenant de la résolution de l'épingle à cheveux des segments réarrangés (Fugmann et al., 2000). La diversité générée n'est pas aussi grande que celle provenant du deuxième mécanisme faisant intervenir l'enzyme terminal deoxynucleotidyl transférase ; 2) la TdT produit une importante diversité, en ajoutant de façon aléatoire des nuclétotides N à l'extrémité 3' de chaque segment codant, sans besoin de matrice génomique (Bogue et al., 1992).

Le mécanisme des réarrangements secondaires contribue à "conserver" de la diversité : la diversité jonctionnelle représente le plus grand facteur d'amplification de la diversité du répertoire, mais s'il n'y avait pas le mécanisme de réarrangement secondaire pour sauver 2/3 des thymocytes ayant interrompu leur cadre de lecture, ce bénéfice en terme de diversité représenterait un coût important pour l'organisme, avant même l'étape de sélection positive. Ces réarrangements non productifs ne peuvent donner une protéine TCR fonctionnelle. La cellule a alors la possibilité de tenter un deuxième réarrangement avec les gènes V(D)J encore disponibles sur le locus. La propriété d'ouverture concentrique du locus TRAD favorise ce processus en laissant le plus de chances possibles à la cellule, puisque les premiers réarrangements effectués par la cellule ont lieu entre un couple de gène V-J proches l'un de l'autre (Pasqual et al., 2002). Si ces premiers réarrangements ne sont pas productifs, la cellule a la possibilité de tenter des réarrangements sur son second chromosome, ou encore d'utiliser les gènes V et J disponibles de part et d'autre du premier réarrangement. Ainsi, les réarrangements secondaires permettent la survie d'un grand nombre de cellules qui, à l'issue d'un premier réarrangement non productif, auraient dû être éliminées.

Les hypermutations somatiques (HMS) ont lieu pendant la différenciation des lymphocytes B dans les ganglions lymphatiques, lors de la rencontre avec un antigène. Les HMS sont situées dans des "*hot spot motifs*" des gènes réarrangés V-J et V(D)J des Ig (Chaudhuri et al., 2003) mais aussi dans certains cas, dans des gènes réarrangés V-J et V(D)J des TCR (Kotani et al., 2005). Le TCR peut être la cible d'HMS au niveau des gènes variables, si le lymphocyte surexprime l'enzyme AID (*Activation-induced cytidine deaminase*) qui est normalement spécifique des lymphocytes B. En temps normal le TCR ne subit pas d'HMS car le lymphocyte T ne synthétise tout simplement pas l'AID. Néanmoins, si le lymphocyte T se met à l'exprimer, le TCR est aussi sensible à cette enzyme que le BCR car il possède toutes les séquences sur laquelle elle agit. Au total, il est décrit dans la littérature que ce mécanisme induit une diversité supplémentaire d'un facteur 1000 dans le but d'augmenter les chances de reconnaître un antigène.

L'estimation de la diversité issue de l'appariement entre une chaîne TCRα et une chaîne TCRβ est obtenue en multipliant le nombre de combinaisons différentes d'une chaîne TCRα, par le nombre de combinaisons possibles pour la chaîne TCRβ. La diversité générée par ce mécanisme est directement dépendante du nombre de combinaisons primaires obtenu lors du réarrangement. En effet, si on examine le nombre de combinaisons primaires TCRγδ chez la souris, sans prendre en compte la diversité jonctionnelle, le résultat est seulement de 40 TCRδ (=10V*2D*2J) x 28 TCRγ (=7V*4J) = 1120 combinaisons différentes, alors que le même calcul conduit à 5,6 10⁶ combinaisons pour TCRαβ (calculé comme suit: 102Vα*60Jα*33Vβ*2Dβ*14Jβ).

La diversité du répertoire des immunoglobulines produites par les lymphocytes B résulte des mêmes mécanismes que ceux décrits ci-dessus pour les lymphocytes T.

La mesure de la diversité immunologique permet entre autres d'étudier les mécanismes de mise en place du répertoire immunitaire, l'homéostasie, les lymphocytes T ou B impliqués dans une réponse immunitaire, dans une leucémie ou encore d'évaluer l'immunodéficience induite par un traitement ou une pathologie, en particulier tumorale, ou à l'inverse l'activation du système immunitaire spécifique. Cette liste n'est pas exhaustive.

L'étude du répertoire immunitaire d'une population lymphocytaire a conduit au développement de plusieurs approches multiparamétriques, permettant à la fois de mesurer le niveau de diversité et d'identifier la présence de certains clones T ou B spécifiques. Certaines approches mises au point par les immunologistes pour évaluer ces différents niveaux de diversité sont listées ci-dessous selon le principe et le "niveau" de diversité mesurée.

### Mesure de la diversité V

- Par cytométrie (Van den Beemd, van Dongen et al. 2000)
- Par Q-PCR au niveau génomique et transcriptomique (Fuschiotti et al., 2007; Pasqual et al., 2002).
- Par séquençage

### Mesure de la diversité jonctionnelle CDR3 :

- Par Immunoscope® (Cochet et al., 1992; Pannetier et al., 1995).
- Q-PCR couplé à l'immunoscope (TcLandscape®)
- Par séquençage
- Par la méthode Amplicot au niveau génomique (Baum and McCune, 2006).
- Par puce à ADN (Bonarius et al., 2006).

### Etude des hypermutations somatiques (HMS) :

- PCR / séquençage (Hamblin et al., 1999).

### Mesure indirecte via la décroissance des cercles d'excisions TREC's

- Par PCR (Douek et al., 1998).
- Par Q-PCR (Pham et al., 2003).

Si certaines de ces approches ont déjà fait leurs preuves en recherche fondamentale, notamment l'Immunoscope® (Pannetier, C., J. Even, et al., 1995) ou la cytométrie en flux (Van den Beemd, van Dongen et al., 2000), il reste encore un certain nombre de validations scientifiques et techniques à apporter pour évaluer la pertinence de leur utilisation comme bio-marqueur médical. Face à la complexité du système immunitaire, le scientifique aurait besoin de coupler des approches technologiques complémentaires pour décrypter l'ensemble des informations contenues dans le répertoire immunitaire et relevantes d'une pathologie donnée.

D'autres méthodes, basées sur l'utilisation de PCR amplifiant spécifiquement des segments d'acides nucléiques caractéristiques de certains réarrangements, ont été décrites. Par exemple, les brevets US 5,296,351 et US 5,418,134 présentent une méthode de détection des leucémies lymphoïdes ou des lymphomes B ou T, basée sur l'amplifications de séquences codant pour des immunoglobulines et/ou des récepteurs T, utilisant des amorces "consensus" pour amplifier simultanément plusieurs réarrangements V-J.

Les inventeurs ont précédemment décrit des méthodes et kits pour mesurer la diversité combinatoire du répertoire des lymphocytes T et/ou B d'un individu (WO 2009/095567). Dans cette demande de brevet, les inventeurs ont également défini la "divpénie" comme un déficit de diversité combinatoire immunitaire, et évoqué le risque de mortalité par infection accru pour des patients en état de divpénie.

Dans le présent texte, la divpénie désigne un déficit de diversité immunitaire, quel que soit le niveau (diversité combinatoire, jonctionnelle ou autre) auquel cette diversité est mesurée. La divpénie T désigne donc un déficit de diversité du répertoire des lymphocytes T.

Wei *et al.* ont caractérisé le changement de répertoire immunologique TCR avant traitement et plusieurs cycles après traitement chez des patients atteints de cancer colorectal (CRC) métastatique (Wei *et al*., 2010). Pour ce faire, ils ont analysé les spectratypes TCR CDR3 de cellules T dans le sang périphérique des patients. Ils ont conclu que leurs résultats démontrent une corrélation positive entre une normalisation d'un répertoire immunologique TCR post-thérapie et une rémission des patients atteints de CRC métastatique, ce qui suggère qu'une caractérisation du répertoire immunologique TCR d'après une analyse de spectratype CDR3 peut avoir une valeur pronostique potentielle.

Dans les études exposées dans la partie expérimentale ci-dessous, les inventeurs ont cherché à déterminer s'il existait une relation entre la divpénie et différents risques à prendre en considération lors du traitement de malades atteints de cancers solides, notamment au stade métastatique. De façon surprenante, ils ont constaté que la divpénie T avait une corrélation directe, forte, avec la mortalité précoce (pas nécessairement par infection), en particulier dans le cas du cancer du sein métastatique (exemple 1). Il est important de noter que la divpénie T n'est pas systématiquement corrélée aux autres marqueurs de mortalité précoce connus, et en particulier à la lymphopénie. En particulier, les inventeurs ont déterminé, dans le cas du cancer du sein métastatique, qu'une diversité combinatoire des réarrangements V(D)J des gènes du locus hTRB inférieure à 20% des réarrangements possibles est un facteur pronostic puissant de décès précoce, avec une médiane de survie inférieure à 6 mois. Ceci ne semble pas être le cas de la divpénie B (exemple 2). En outre, contrairement à ce qui aurait pu être attendu, la divpénie (T ou B) ne semble pas être un facteur de risque de toxicité grave des traitements chimiothérapeutiques utilisés pour le traitement du cancer du sein primaire (résultats non montrés).

L'identification rapide, et avant tout traitement, de patients ayant un risque accru de mortalité précoce a des conséquences importantes pour ces patients et pour la recherche médicale, car elle permet d'envisager un suivi particulier pour ces malades, le cas échéant avec une hospitalisation plus soutenue et/ou l'administration de traitements moins immunosuppresseurs ou plus ciblés sur la stimulation de leur système immunitaire. Pour la recherche médicale, elle permet d'identifier une population de patients homogène pour laquelle le traitement de référence sera vraisemblablement inefficace et pour laquelle les cliniciens semblent actuellement particulièrement démunis. La caractérisation d'une telle population représente un enjeu majeur pour les cliniciens et les industries pharmaceutiques pour les essais cliniques de traitements innovants qui auront une efficacité supérieure à celle des traitements de référence, augmentant ainsi la possibilité d'enregistrement de nouveaux médicaments. Cette population pourra être de préférence sélectionnée pour effectuer des essais cliniques pour tester des traitements innovants.

La présente invention porte donc en premier lieu sur l'utilisation de la diversité du répertoire des lymphocytes T d'un individu atteint d'un cancer solide métatstatique, comme marqueur pronostique de l'évolution de ce cancer. En particulier, en cas de divpénie T, ce marqueur est indicatif d'un risque accru de décès précoce.

Plus particulièrement, la présente invention concerne une méthode permettant de déterminer *ex vivo* ou *in vitro* si un patient atteint d'un cancer solide a un risque accru de décès précoce, autrement dit pour établir *ex vivo* ou *in vitro* un pronostic pour un individu souffrant d'un cancer solide métatstatique, comprenant les étapes suivantes :
(i) à partir d'acide nucléique (par exemple, d'ADN génomique ou d'ARN messager) provenant d'un échantillon biologique contenant des lymphocytes dudit individu, prélevé avant l'administration de la première ligne de chimiothérapie, mesurer le niveau de diversité du répertoire des lymphocytes T dudit individu ;
(ii) comparer le niveau de diversité mesuré à l'étape (i) à un seuil prédéterminé ;
(iii) en déduire, dans le cas où le niveau de diversité mesuré à l'étape (i) est inférieur au seuil prédéterminé, que l'individu a un risque élevé de décès précoce.

Un exemple particulier de cancer solide pour lequel cette méthode est appropriée est le cancer du sein en phase métatstatique, mais en se basant sur les travaux précédents des inventeurs sur la lymphopénie (Borg et al., 2004; Ray-Coquard et al., 2009) elle peut également être transposée à tous les cancers solides, tels que les cancers de la prostate, du poumon, du côlon, ovariens, de la sphère ORL, les lymphomes, les sarcomes, *etc*, lorsqu'ils deviennent métastatiques.

Bien évidemment, le seuil considéré à l'étape (ii) peut dépendre du profil clinique du patient (type et stade de cancer, et le cas échéant âge et autres paramètres physiologiques), et l'homme du métier est à même de conduire les expérimentations nécessaires, par des études rétrospectives ou prospectives, pour définir un seuil pertinent pour un type de pathologie et/ou de patient donné.

De même, la notion de "mortalité précoce" est à rapporter au type de cancer dont est atteint l'individu, ainsi qu'au stade d'avancement de ce cancer (notamment, savoir s'il est métastatique ou non). On parlera ici de "risque accru (ou élevé) de mortalité précoce" pour un patient lorsqu'il appartient, dans une cohorte représentative de son état pathologique, à une sous-population dont la médiane de survie est inférieure à celle de la cohorte entière (un exemple de cohorte représentative de patients atteints de cancer du sein métastatique est donné dans la partie expérimentale). Pour une sous-population donnée (par exemple, patients en état de divpénie T), on parlera de mortalité précoce lorsque la médiane de survie de cette sous-population (représentative de l'espérance de vie des membres de cette sous-population) est statistiquement significativement inférieure à celle de la cohorte entière. Dans le cadre de la présente invention, la mortalité précoce des malades en état de divpénie T correspond typiquement à une espérance de vie deux ou trois fois moindre, voire 5 fois moindre que celle d'une population de malades atteints du même cancer au même stade, sans qu'il soit tenu compte de leur niveau de diversité immunitaire (ou, *a fortiori*, que celle d'une population de malades atteints du même cancer au même stade, mais ayant une diversité lymphocytaire satisfaisante). Ainsi, un individu ayant un risque accru de décès précoce a une espérance de vie significativement inférieure (typiquement, deux fois moindre) que celle qu'il aurait avec le même bilan médical et biologique (type et stade du cancer, PS, taux d'hémoglobine *etc*.), sans tenir compte de sa diversité lymphocytaire T, ou si celle-ci était supérieure à un seuil déterminé (par exemple, supérieure à 40%).

Un autre paramètre susceptible de jouer sur le seuil de diversité des lymphocytes T en dessous duquel la divpénie T constitue un marqueur significatif de risque de mortalité précoce est la technologie avec laquelle est mesurée cette diversité. En effet, comme mentionné plus haut, l'homme du métier dispose d'un grand nombre de technologies (séquençage, immunoscope, puce à ADN, ...) pour mesurer, à différents niveaux, la diversité du répertoire des lymphocytes T d'un individu. La présente invention peut être mise en œuvre en utilisant n'importe quelle technologie mesurant la diversité des lymphocytes T, quel que soit le matériel d'origine (ADNg, ARN, etc...) et le niveau auquel est mesurée cette diversité (diversité combinatoire, jonctionnelle, *etc*.). La transposition des résultats décrits dans la partie expérimentale ci-dessous, par des expérimentations et une analyse statistique de routine, permettra de déterminer un seuil significatif pour la technologie alternative utilisée.

Selon un mode de réalisation particulier de l'invention, illustré expérimentalement ci-dessous, la diversité est mesurée au niveau combinatoire. Les inventeurs, dans la demande de brevet publiée sous le numéro WO 2009/095567, ont décrit plusieurs méthodes de mesure de la diversité combinatoire, selon le(s) locus ciblé(s) (TRA, TRB, TRG, TRD), l'analyse ou non des réarrangements incomplets, le pourcentage, pour chaque locus, de réarrangements analysés (en effectuant un nombre plus ou moins important de PCR), le niveau d'analyse de ces réarrangements (détection du pourcentage de réarrangements ou quantification de chaque réarrangement observé et précisément identifié), *etc.*

Dans le cadre de l'étude expérimentale exposée ci-dessous, la diversité combinatoire du répertoire des lymphocytes T a été évaluée à partir de l'analyse des réarrangements V(D)J du seul locus TRB, par une méthode permettant d'analyser plus de 80% des réarrangements V(D)J du locus TRB. Une méthode dans laquelle la technologie utilisée à l'étape (i) permet d'analyser au moins 70% des réarrangements V(D)J du locus TRB constitue d'ailleurs un mode de mise en œuvre préféré de l'invention. En particulier, la méthode de l'invention peut être avantageusement mise en œuvre en effectuant la mesure de la diversité combinatoire du répertoire des lymphocytes T par des PCR multi-n-plexes avec n≥2 à l'aide de combinaisons d'au moins 3 amorces, chaque combinaison d'amorces comprenant au moins les amorces hTRBJ1.6 (CTTGGTGCATGGCTATGTAATCCTG, SEQ ID No : 1), hTRBJ2.7 (CTCGCCCTCTGCTCAGCTTTCC, SEQ ID No : 2) et une amorce hTRBV choisie dans le groupe constitué des amorces de SEQ ID Nos : 3 à 25. Une méthode selon l'invention, dans laquelle l'analyse du locus TRB est réalisée en effectuant au moins 23 PCR multi-2-plexes, chaque PCR multi-2-plexe étant réalisée avec un triplet d'amorces constitué des amorces hTRBJ1.6 (SEQ ID No : 1), hTRBJ2.7 (SEQ ID No : 2) et d'une amorce hTRBV choisie dans le groupe constitué des amorces de SEQ ID Nos : 3 à 25 (Tableau 1), constitue un mode de mise en œuvre préféré de l'invention.

**Tableau 1 : amorces s'hybridant au locus TRB (gènes V), utilisables dans le cadre de la présente invention**

| Nom du gène | Nom de l'amorce | Taille (nt) | Distance entre l'extrémité 5' de l'amorce et la fin du gène V (pb) | Séquence | SEQ ID N° |
|---|---|---|---|---|---|
| TRBV2 | hTRBV2up2 | 26 | 255 | CACACAGATGGGAC AGGAAGTGATCT | 3 |
| TRBV4 | hTRBV4up_ex | 23 | 100 | GCTTCTCACCTGAAT GCCCCAAC | 4 |
| TRBV5.1, 3, 4, 5, 6, 8 | hTRBV5up_ex1/2 | 25 | 256 | CTGATCAAAACGAG AGGACAGCAAG | 5 |
| TRBV5.7 | hTRBV5up_ex2/2 | 25 | 256 | CTGATCAAAACGAG AGGACAGCACG | 6 |
| TRBV6.4 | hTRBV6up_ex2/2 | 23 | 279 | GATCACCCAGGCAC CAACATCTC | 7 |
| TRBV7.2 | hTRBV7up_ex2/3 | 25 | 301 | CAGATCACACAGGA GCTGGAGTCTC | 8 |
| TRBV7.9 | hTRBV7up_ex3/3 | 27 | 303 | CACAGATCACGCAG ATACTGGAGTCTC | 9 |
| TRBV9 | hTRBV9up_ex | 23 | 92 | CGCACAACAGTTCCC TGACTTGC | 10 |
| TRBV11 | hTRBV11up_ex | 27 | 120 | TTCACAGTTGCCTAA GGATCGATTTTC | 11 |
| TRBV12.1 | hTRBV12.1up1 | 27 | 196 | TTCTCTGGTACAGAC AGACCTTTGTGC | 12 |
| TRBV12.2 | hTRBV12.2up1 | 27 | 196 | TTTTCTGGTACAGAG ATACCTTCGTGC | 13 |
| TRBV13 | hTRBV13up1 | 25 | 356 | GTTGCTGAAGTGTCA AACTCTCCCG | 14 |
| TRBV14 | hTRBV14up_ex | 24 | 271 | TCCCCAGCCACAGC GTAATAGAGA | 15 |
| TRBV15 | hTRBV15up_ex | 24 | 163 | CCCCAAAGCTGCTGT TCCACTACT | 16 |
| TRBV16 | hTRBV16up1 | 22 | 295 | CTCCTGGTGAAGAA GTCGCCCA | 17 |
| TRBV18 | hTRBV18up1 | 22 | 46 | TAGTGCGAGGAGAT TCGGCAGC | 18 |
| TRBV19 | hTRBV19 up 2 | 24 | 217 | CTGGGAGCAAGTGA GTCCTGGGT | 19 |
| TRBV20 | hTRBV20-1up_ex | 24 | 91 | TCATCAACCATGCAA GCCTGACCT | 20 |
| TRBV24 | hTRBV24up_ex | 24 | 96 | AGTGTCTCTCGACAG GCACAGGCT | 21 |
| TRBV25 | hTRBV25up_int | 23 | 273 | CCTCTTTGTTGGGTT TGTGCCTG | 22 |
| TRBV27 | hTRBV27up2 | 22 | 312 | GTCCCCTTCCTTTAC AGGCCCC | 23 |
| TRBV29 | hTRBV29up_G | 21 | 91 | CCATCAGCCGCCCA AACCTAA | 24 |
| TRBV30 | hTRBV30up1 | 26 | 148 | TGCTCTTCTACTCCG TTGGTATTGGC | 25 |

Le cas échéant, l'homme du métier peut choisir d'évaluer la diversité combinatoire du répertoire des lymphocytes T en examinant un nombre plus limité de réarrangements V(D)J du locus TRB, et/ou en examinant des réarrangements d'un autre locus choisi parmi les locus TRA, TRG et TRD. Le pourcentage de diversité du répertoire T du patient sera alors extrapolé, en calculant le pourcentage de réarrangements observés parmi les réarrangements théoriquement observables avec la technologie utilisée. Bien entendu, le résultat obtenu sera d'autant plus informatif que la technologie utilisée permettra l'observation théorique d'un nombre élevé de réarrangements. Le coût de l'analyse sera donc mis en balance avec le niveau d'information souhaité, selon les situations, pour déterminer un nombre optimum de réarrangements observables. En tout état de cause, il est préférable, pour obtenir un résultat exploitable, d'analyser au moins 10 réarrangements, de préférence au moins 20, ou 30, voire 50 réarrangements du locus TRA ou du locus TRB. De manière encore préférée, on utilisera une technologie qui permet d'observer au moins 20% des réarrangements théoriques possibles d'un de ces locus (299 réarrangements V-J pour le locus TRB et 2500 pour le locus TRA).

L'exemple 5 ci-dessous présente une technologie alternative à la technologie utilisée dans les autres exemples pour mesurer la diversité du répertoire des lymphocytes T. Dans cet exemple, la diversité moléculaire (associant la diversité combinatoire et la diversité du CDR3) est mesurée par séquençage à haut débit d'ADN de PBMC, selon la technique décrite par Robins *et al.* (Robins et al., 2009). Cet exemple montre que la diversité du répertoire immunitaire peut être mesurée par différentes technologies, en particulier par séquençage ; la diversité du répertoire immunitaire demeure, quelle que soit la technologie utilisée pour la mesurer, un marqueur pronostique pour les cancers solides (dès lors que cette mesure est suffisamment quantitative et qualitative).

Pour la mise en œuvre de l'invention, l'ADN génomique est de préférence purifié. Toutefois, l'homme du métier peut, en fonction de l'évolution des technologies, choisir de travailler sur des échantillons bruts. Tout échantillon biologique susceptible de contenir des lymphocytes T peut être utilisé ; à titre d'exemples non limitatifs d'échantillons utilisables, on peut citer des échantillons de sang (sang total ou PBMC par exemple), thymus, ganglion, rate, sein, foie, peau, ou plus généralement tout échantillon tumoral, ainsi qu'un fluide biologique tel qu'un épanchement pleural ou un ascite.

Selon une mise en œuvre préférée de l'invention, le seuil de diversité immunitaire auquel sera comparée la diversité d'un patient atteint de cancer métastatique sera prédéterminé de façon à ce que l'espérance de survie d'un patient dont le niveau de diversité est inférieur à ce seuil est au moins deux fois moindre que l'espérance de survie généralement observée pour les patients atteints du même cancer solide métastatique que celui dont il est atteint.

Selon une mise en œuvre particulière de l'invention, illustrée dans l'exemple 1 sur une cohorte de patientes atteintes de cancer du sein métastatique, le seuil considéré à l'étape (ii) du procédé est fixé à 33% ou à 30% de diversité, et l'interprétation à l'étape (iii) consiste à dire que si le niveau de diversité combinatoire des lymphocytes T mesuré est inférieur à ce seuil, l'individu a une espérance de vie deux fois moindre que celle généralement observée pour sa pathologie. Bien évidemment, les réserves exposées plus haut restent pertinentes, et l'homme du métier pourra, sur un type ou un stade différent de cancer, et/ou en utilisant une technologie différente de l'analyse de la diversité combinatoire des réarrangements du locus TRB, ajuster le seuil, de façon à obtenir un seuil de diversité en deçà duquel l'espérance de vie des patients est deux fois moindre que pour les patients atteints de la même pathologie, indépendamment de leur statut immunologique.

Selon une autre mise en œuvre particulière de l'invention, également illustrée à l'exemple 1, le seuil considéré à l'étape (ii) du procédé est fixé 25% ou à 20% de diversité, et l'interprétation à l'étape (iii) consiste à dire que si le niveau de diversité combinatoire des lymphocytes T mesuré est inférieur à ce seuil, l'individu a une espérance de vie cinq fois moindre que celle généralement observée pour sa pathologie. L'invention porte plus spécifiquement sur une méthode telle que décrite ci-dessus, dans laquelle le patient est atteint d'un cancer du sein métastatique, et dans laquelle un niveau de diversité (en particulier, de diversité combinatoire) des réarrangements V(D)J du locus TRB inférieur à 20-25% est indicatif d'une espérance de survie du patient inférieure à 6 mois (p= 2.10⁻⁷).

Il est particulièrement intéressant de noter que le marqueur objet de la présente invention est indépendant des autres facteurs de risque identifiés à ce jour (p= 0.0092). En conséquence, la méthode selon l'invention peut être mise en œuvre pour établir un pronostic sans prendre en compte ces marqueurs, en particulier sans prendre en compte la numération lymphocytaire du patient. La divpénie T peut toutefois, en accord avec l'invention, être combinée avec d'autres paramètres, immunitaires tels que le niveau de cytokines sériques, en particulier de type IL7 ou IL15, ou le nombre de cellules CD4+, ou encore avec d'autres paramètres biologiques ou cliniques tels que l'âge, le *performance status*, la numération lymphocytaire, le nombre de cellules CD4+ ou le niveau d'hémoglobine, pour établir un pronostic chez un patient atteint d'un cancer solide. Comme illustré à l'exemple 4 ci-dessous, la combinaison de la numération et de la diversité lymphocytaires permet de ségréger les patients de façon beaucoup plus précise que l'utilisation d'un seul de ces marqueurs. Elle permet d'identifier une sous-population particulièrement à risque (zone 1 du graphique NDL, dite de « lymphodivpénie » pour signifier que les individus ont une faible quantité de lymphocytes, présentant en outre une diversité insuffisante).

Un autre aspect de la présente invention est de sélectionner des patients à risque pour les inclure dans des protocoles cliniques innovants ayant pour objectifs soit de corriger la lymphopénie et/ou la divpénie (cytokines type IL2, IL7, IL15, immunostimulation, compléments alimentaires,...) soit d'évaluer des thérapies émergentes. La possibilité d'identifier les patients à risque avant tout traitement de phase métastatique notamment, est également susceptible d'augmenter fortement les chances de bénéfice du traitement par rapport au risque, et donc de valider une thérapie innovante par rapport à la thérapie de référence. Dans certains cas, cette approche de stratification des patients permet en parallèle de réduire le coût de l'étude clinique en ne traitant que les patients à risque (un exemple de comparaison des coûts est donné à l'exemple 3 ci-dessous, à titre purement indicatif).

L'invention porte donc sur une méthode pour déterminer *ex vivo* ou *in vitro* si un patient atteint d'un cancer solide doit être inclus dans un protocole de recherche clinique pour tester un nouveau médicament, comprenant les étapes suivantes :
(i) déterminer, en mettant en œuvre une méthode de pronostic telle que décrite plus haut, si le patient a un risque accru de décès précoce, et
(ii) si le patient a un risque accru de décès précoce, l'inclure dans le protocole de recherche clinique.

L'identification, par une méthode telle que décrite ci-dessus, de patients particulièrement à risque, qui ne répondront vraisemblablement pas bien à une thérapeutique conventionnelle ou de référence, permet en outre d'adapter le traitement de ces patients, par exemple pour leur proposer un suivi hospitalier particulier, une antibiothérapie préventive, une immunostimulation médicamenteuse ou par des compléments alimentaires, une vaccination thérapeutique, une chimiothérapie adaptée, de préférence la moins immunosuppressive possible, ou encore un changement de posologie ou de fréquence d'administration d'une chimiothérapie qu'ils reçoivent déjà.

Outre les dispositions qui précèdent, l'invention comprend encore d'autres dispositions, qui ressortiront des exemples expérimentaux ci-dessous, et des figures annexées.

### Légende des figures

**Figure 1** : Courbes de survie des patientes selon leur diversité hTRVJ. Survie globale avec un seuil de diversité combinatoire TCR de **A)** 20% (p=2.10⁻⁷) ; **B)** 30% (p=0,0047) ; **C)** 33% ; **D)** 40% (p=0,481).

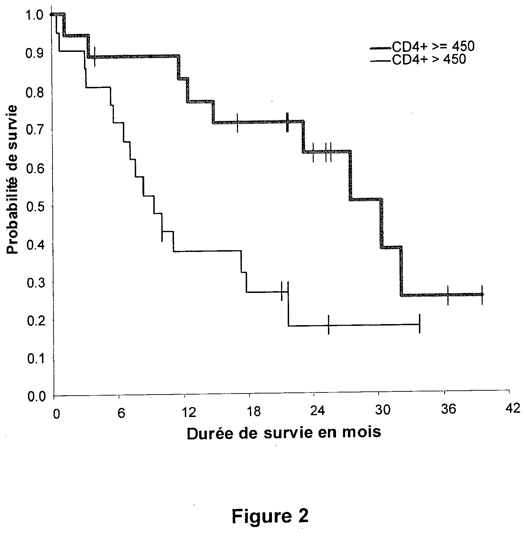

**Figure 2** : Survie globale en fonction de la quantité de lymphocytes T CD4+ circulants (en gris épais patientes avec CD4+>450/µl et en noir CD4+≤450/µl) (p=0.011).
**Figure 3** **:** Courbes ROC pour déterminer les patientes à risque de décès précoce (≤ 12 mois) dans la cohorte WP1a en utilisant comme marqueur **A)** la divpénie ou **B)** la lymphopénie.
**Figure 4****:** Etude sur la cohorte WP1a. **A)** graphique NDL; **B)** Analyse de la survie selon Kaplan Meyer en comparant les patients de la zone NDL1 aux patients des trois autres zones ; **C)** Analyse de la survie selon Kaplan Meyer en fonction de leur état lymphopénique ou non (seuil à 0,7 Giga/l).
**Figure 5****:** Etude sur la cohorte WP1b. **A)** graphique NDL ; **B)** Analyse de la survie selon Kaplan Meyer en comparant les patients de la zone NDL1 aux patients des trois autres zones.
**Figure 6** : Schéma de la technologie utilisée à l'exemple 5.
**Figure 7****:** Représentation 3D de la diversité d'un échantillon de PBMC de sujet sain, mesurée **A)** et **B)** par séquençage, et **C)** par PCR multi-N-plexes.
**Figure 8****:** Représentation 3D de la diversité d'un échantillon de PBMC de sujet leucémique T, mesurée **A)** et **B)** par séquençage, et **C)** par PCR multi-N-plexes.

### EXEMPLES

### Exemple 1 : Etude de la corrélation entre un risque accru de décès précoce et une faible diversité combinatoire T, chez des patientes atteintes de cancer du sein métastatique

### Matériels et méthodes

### Sélection des patientes

Patientes incluses dans le protocole clinique SEMTOF mené au CLB (Centre Léon Bérard) : patientes atteintes d'un cancer du sein phase métastatique en 1ère ligne de chimiothérapie dont le PS (*Performance Status*) est inférieur ou égal à 2. Les patientes inclues ont reçu les traitements de chimiothérapie classiquement utilisés dans cette pathologie. Le prélèvement sanguin sur lequel l'analyse du répertoire immunitaire sera effectuée, est réalisé avant l'administration de la première ligne de chimiothérapie.

### Mesure de la diversité combinatoire T

Une PCR Multiplexe ImmunTraCkeR®β est réalisée en utilisant un oligonucléotide "*upstream*" spécifique de tous les membres d'une famille V donnée et un oligonucléotide "*downstream*" spécifique d'une famille J donnée. Cette technologie permet la détection simultanée de plusieurs réarrangements V-J dans la même réaction. Le test ImmunTraCkeR®β est composé de 23 puits (+1 puits pour le contrôle qualité interne), chacun capable de détecter l'ensemble des réarrangements d'une famille V. Ce test permet de détecter 276 réarrangements hTRB V-J différents (276 réarrangements observés correspondraient donc ici à 100% des réarrangements observables). De la même manière, il est possible de détecter les 48 réarrangements hIgH V-J possible, assurant une couverture complète de ce répertoire. Les conditions de PCR ont été décrites précédemment (Marodon et al., 2009).

La description de l'estimation semi-quantitative et qualitative du répertoire est décrite ci-dessous.

Attribution des réarrangements : cette attribution consiste à rapprocher la taille des produits de PCR mesurée avec un standard dont la taille et la concentration de chaque produit sont connues. Cette analyse s'effectue soit de façon manuelle soit grâce au logiciel Constel'ID développé par ImmunID.

Evaluation semi quantitative : La PCR est arrêtée en fin de phase exponentielle d'amplification. Le signal est mesuré en fonction de l'intensité de fluorescence de notre marqueur par une caméra CCD du type puce à ADN. La quantification du signal numérique est assurée par un logiciel d'acquisition qui mesure l'intensité de fluorescence des réarrangements V-J détectés, normalisée par rapport au standard de migration.

Evaluation qualitative du répertoire immunitaire : un score de diversité du répertoire est calculé en fonction du nombre maximum de réarrangements attendus (nombre de réarrangements V(D)J présents dans l'échantillon, divisé par le nombre de réarrangements théoriquement observables avec le kit ImmunTraCkeR (en l'occurrence, 276), multiplié par 100). Ces données permettent d'évaluer l'aspect qualitatif du répertoire immunitaire et d'estimer le niveau de perturbation du répertoire suite à un traitement.

### Analyses statistiques

Afin de déterminer le rôle des différents biomarqueurs du cancer dans la survie des patientes, un modèle d'estimation de la survie globale a été construit. Le type de modèle statistique utilisé pour ce faire est le modèle de Cox. La construction du modèle est réalisée sur la base de la mise en présence de différents facteurs pronostiques déjà identifiés pouvant être protecteurs ou à risque de décès du cancer (ex : une faible concentration d'hémoglobine est à risque pour la patiente). La valeur prédictive de chacun de ces différents facteurs est préalablement évaluée individuellement (analyse univariée), afin de déterminer la pertinence de leur intégration dans un modèle multifactoriel (analyse multivariée) de prédiction de la survie globale. Cette évaluation préalable est aussi réalisée par l'application d'un modèle de Cox.

### Courbe de Kaplan Meier

La méthode de Kaplan-Meier permet d'estimer la probabilité de survie avec son intervalle de confiance à 95 % pour des données censurées à droite et de tracer des courbes de survie (Kaplan and Meier, 1958; Rothman, 1978). Les intervalles de temps débutent à l'instant t où survient un décès et se terminent juste avant le décès suivant.

### Modèle de Cox

Le modèle de Cox prend en compte l'effet de facteurs confondants explicatifs de la survie par une analyse dite multivariée (Cox, 1972; Therneau and Grambsch, 2000). L'effet d'une variable sur la survie est modélisé après ajustement sur les autres variables explicatives de décès introduites dans le modèle.

Survie globale : la survie globale a été définie comme la date d'entrée dans le protocole jusqu'à la mort de la patiente ou la date de dernières nouvelles pour les patientes toujours en vie au dernier contact.

### Résultats

L'étude de la diversité combinatoire du TCR au moment de la rechute chez des patientes atteintes d'un cancer du sein permet de prédire le risque de décès précoce (< 6 mois) et ainsi d'identifier un sous-groupe de patientes vraisemblablement réfractaires au traitement de référence et donc éligibles pour l'accès à des innovations thérapeutiques dans le cadre d'essais cliniques.

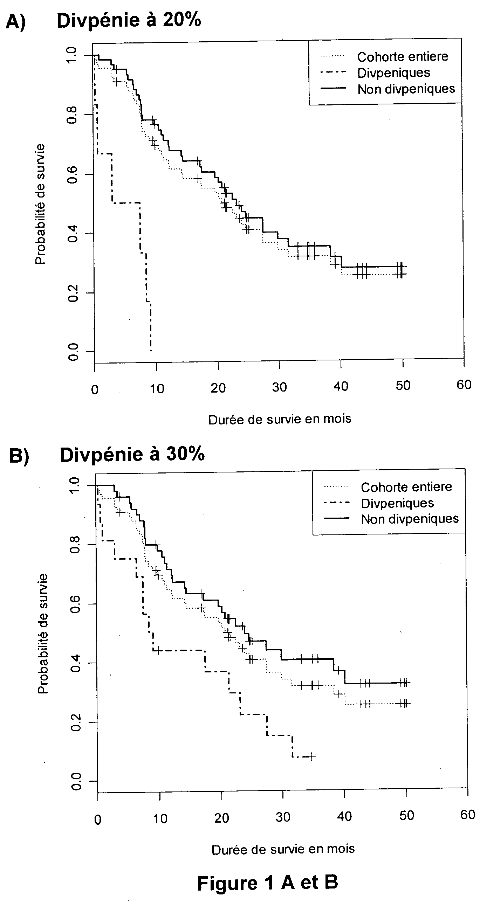

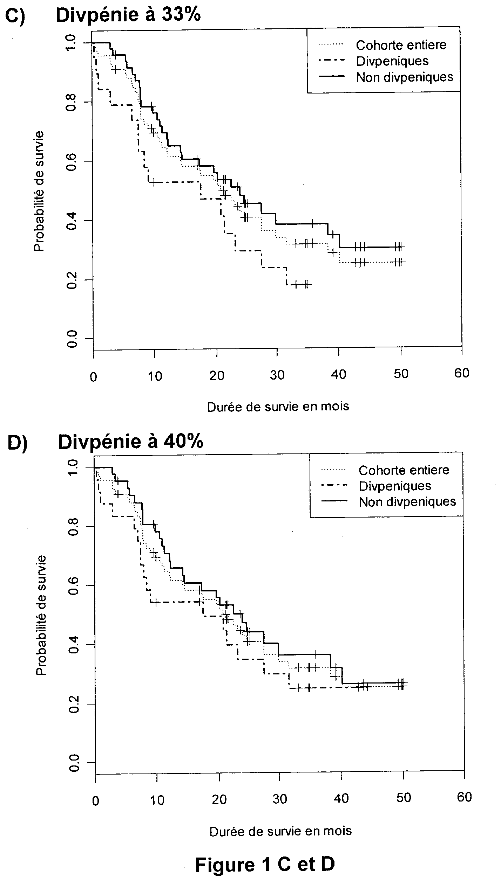

Une étude statistique univariée réalisée sur une cohorte de 66 patientes a montré qu'une diversité combinatoire de la chaîne β du TCR <20% est un marqueur d'un risque de décès précoce (Fig.1-C). Ce marqueur est significatif dès le seuil de diversité de 30% (Fig. 1B) et est optimum à 20% (Fig.1C). 6/6 patientes à diversité <20% sont décédées avec une médiane de survie de 5.21 mois *vs.* 37/60 décès pour les patientes à diversité >20% avec une médiane de survie de 23.2 mois.

Une analyse multivariée confirme les résultats observés en univarié lorsque la divpénie est intégrée dans un modèle de prédiction simple validé (Tableau 2). Une diversité combinatoire hTRB <20% est un facteur indépendant (p-value <0.05) des autre facteurs pronostiques tels que le taux d'hémoglobine, le taux de PNN et la localisation hépatique des métastases. Notons que la lymphopénie (LT<700/µl) n'est pas apparue comme un facteur pronostique de décès précoce dans l'ensemble de la cohorte étudiée (P=0.35).

**Tableau 2 analyse multivariée de la diversité combinatoire TRB seuil 20% (valeur qualitative) en fonction de la survie globale (HR : Hazard Ratio ; SD: Standard Deviation)**

| **FACTEURS** | **groupes** | **Taux de survie à 9 mois** | **HR** | **SD** | **P** | **IC inf 95%** | **IC sup 95%** |
|---|---|---|---|---|---|---|---|
| **Hémoglobine (g/dL)** | **< 11,5** | **44%** | **3,285** | **0,369** | **1,3.10⁻³** | **1,592** | **6,776** |
| | **>= 11,5** | **78%** | | | | | |
| **PNN (Giga/L)** | **<= 7,5** | **70%** | **1,821** | **0,567** | **0,290** | **0,599** | **5,531** |
| | **> 7,5** | **57%** | | | | | |
| **Site tumoral méta hépatiques** | **Non** | **[82%;88%]** | **0,338** | **0,450** | **0,016** | **0,140** | **0,817** |
| | **Oui** | **64%** | | | | | |
| **DIVPENIE.TRB.VJ** | **< 20%** | **17%** | **4,743** | **0,598** | **0,009** | **1,470** | **15,301** |
| | **>= 20%** | **78%** | | | | | |

Il est communément admis que la mesure du nombre de PNN est un des marqueurs de risque des patients atteints de cancers ou autres pathologies infectieuses.

La lymphopénie CD4+ décrite comme un marqueur de décès précoce (Borg et al., 2004) indique que le système immunitaire, et plus particulièrement les lymphocytes T, peuvent présenter un intérêt dans le pronostic de décès précoce. Or, de manière tout à fait intéressante, l'analyse statistique multivariée avec comme référentiel une évaluation du taux de survie à 9 mois indique que la divpénie TRB <20% est un facteur pronostique plus puissant que la mesure des PNN et de la présence de métastases hépatiques. Par ailleurs, les observations des inventeurs ont montré que la mesure de la divpénie TRB est un facteur indépendant de la mesure de la lymphopénie CD4+ (p-value <0.05) (tableaux 3 & 4).

**Tableau 3 : analyse multivariée de la diversité combinatoire TRB (seuil 30%) versus numération CD4 (seuil 450 cellules/µl) (valeur qualitative) en fonction de la survie globale.**

| **FACTEUR** | **groupes** | **Taux de survie à 14 mois** | **HR** | **SD** | **p** | **IC inf 95%** | **IC sup 95%** |
|---|---|---|---|---|---|---|---|
| **CD4+** | **<= 0,450** | **28%** | **2,503** | **0,461** | **4,7.10⁻²** | **1,014** | **6,183** |
| | **>0,450** | **71%** | | | | | |
| **DIVPENIE_TRB_VJ** | **<30%** | **36%** | **2,232** | **0,431** | **6,3.10⁻²** | **0,959** | **5,198** |
| | **>= 30%** | **61%** | | | | | |

**Tableau 4 analyse multivariée de la diversité combinatoire TRB (seuil 20%) versus numération CD4 (seuil 450 cellules/µL) (valeur qualitative) en fonction de la survie globale.**

| **FACTEUR** | **groupes** | **Taux de survie à 9 mois** | **HR** | **SD** | **P** | **IC inf 95%** | **IC sup 95%** |
|---|---|---|---|---|---|---|---|
| **CD4+** | **<= 0,450** | **45%** | **2,489** | **0,461** | **0,048** | **1,008** | **6,148** |
| | **>0,450** | **[83%;88%]** | | | | | |
| **DIVPENIE_TRB VJ** | **<20%** | **17%** | **5,188** | **0,597** | **0,006** | **1,611** | **16,709** |
| | **>=20%** | **78%** | | | | | |

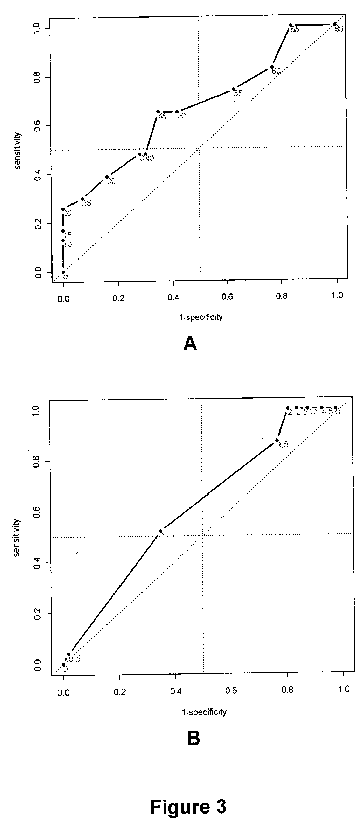

La figure 3 présente les courbes ROC (Receiver Operating Characteristic) pour la cohorte étudiée dans cet exemple, en distinguant les patientes décédées avant et après 12 mois, en fonction de la diversité lymphpcytaire (courbe 3A) ou de la numération lymphocytaire (courbe 3B).

Ces courbes ont été réalisées à partir des données suivantes :

**Tableau 5 : données pour réaliser la courbe ROC en fonction de la divpénie**

| seuils | VP | VN | FP | FN | Se | Sp | 1-Sp |
|---|---|---|---|---|---|---|---|
| 0 | 0 | 43 | 0 | 23 | 0 | 1 | 0 |
| 5 | 0 | 43 | 0 | 23 | 0 | 1 | 0 |
| 10 | 3 | 43 | 0 | 20 | 0,13 | 1 | 0 |
| 15 | 4 | 43 | 0 | 19 | 0,17 | 1 | 0 |
| 20 | 6 | 43 | 0 | 17 | 0,26 | 1 | 0 |
| 25 | 7 | 40 | 3 | 16 | 0,3 | 0,93 | 0,07 |
| 30 | 9 | 36 | 7 | 14 | 0,39 | 0,84 | 0,16 |
| 35 | 11 | 31 | 12 | 12 | 0,48 | 0,72 | 0,28 |
| 40 | 11 | 30 | 13 | 12 | 0,48 | 0,7 | 0,3 |
| 45 | 15 | 28 | 15 | 8 | 0,65 | 0,65 | 0,35 |
| 50 | 15 | 25 | 18 | 8 | 0,65 | 0,58 | 0,42 |
| 55 | 17 | 16 | 27 | 6 | 0,74 | 0,37 | 0,63 |
| 60 | 19 | 10 | 33 | 4 | 0,83 | 0,23 | 0,77 |
| 65 | 23 | 7 | 36 | 0 | 1 | 0,16 | 0,84 |
| 70 | 23 | 0 | 43 | 0 | 1 | 0 | 1 |
| 75 | 23 | 0 | 43 | 0 | 1 | 0 | 1 |
| 80 | 23 | 0 | 43 | 0 | 1 | 0 | 1 |

**Tableau 6 : données pour réaliser la courbe ROC en fonction de la lymphopénie**

| thresholds | VP | VN | FP | FN | Se | Sp | 1-Sp |
|---|---|---|---|---|---|---|---|
| 0 | 0 | 43 | 0 | 23 | 0 | 1 | 0 |
| 0,5 | 1 | 42 | 1 | 22 | 0,04 | 0,98 | 0,02 |
| 1 | 12 | 28 | 15 | 11 | 0,52 | 0,65 | 0,35 |
| 1,5 | 20 | 10 | 33 | 3 | 0,87 | 0,23 | 0,77 |
| 2 | 23 | 8 | 35 | 0 | 1 | 0,19 | 0,81 |
| 2,5 | 23 | 7 | 36 | 0 | 1 | 0,16 | 0,84 |
| 3 | 23 | 5 | 38 | 0 | 1 | 0,12 | 0,88 |
| 3,5 | 23 | 5 | 38 | 0 | 1 | 0,12 | 0,88 |
| 4 | 23 | 3 | 40 | 0 | 1 | 0,07 | 0,93 |
| 4,5 | 23 | 3 | 40 | 0 | 1 | 0,07 | 0,93 |
| 5 | 23 | 1 | 42 | 0 | 1 | 0,02 | 0,98 |

L'aire sous la courbe (AUC) est de 0,67 pour la divpénie et de 0,63 pour la lymphopénie, ce qui confirme que la divpénie est un facteur de risque plus puissant que la lymphopénie.

### Conclusion

Le kit ImmunTraCkeR β permet de prédire les risques de décès précoce chez les patientes atteintes de cancer du sein en situation métastatique. Ce marqueur est indépendant de la lymphopénie CD4+ (<450 CD4+/µl) décrite par Borg C & al. (2004).

### Exemple 2 : Etude de la corrélation entre un risque de décès précoce accru et une faible diversité combinatoire B, chez des patientes atteintes de cancer du sein métastatique

### Matériels et méthodes

La diversité combinatoire des lymphocytes B a été étudiée en utilisant le test hIgH®.

Une PCR Multiplexe IgH® est réalisée en utilisant un oligonucléotide "*upstream*" spécifique de tous les membres d'une famille V donnée et un oligonucléotide "*downstream*" spécifique d'une famille J donnée. Cette technologie permet la détection simultanée de plusieurs réarrangements V-J dans la même réaction. Le test IgH® est composé de 8 puits chacun capable de détecter l'ensemble des réarrangements d'une famille V. Ce test permet de détecter 48 réarrangements hIgH V-J différents (donc l'observation de 48 réarrangements correspond à 100% des réarrangements observables). Les conditions de PCR sont décrites dans l'article de Gilles Marodon et al. 2009, *supra.*

Les autres matériels et méthodes sont identiques à ceux utilisés à l'exemple 1 ci-dessus.

### Résultats

La quantité de lymphocytes B a été étudiée auparavant (Borg & al. 2004), sans être identifiée en tant que marqueur de décès précoce chez les patientes atteintes de cancer du sein métastatique. La corrélation entre la diversité combinatoire IgH et la survenue d'un décès précoce a néanmoins été étudiée sur la même cohorte de patientes que celle décrite à l'exemple précédent (66 patientes).

Une analyse univariée de la survie globale en fonction de la divpénie IgH (tableau 5) n'a pas permis de mettre en évidence de résultat significatif (p-value >5%).

**Tableau 7: analyse univariée de la diversité combinatoire (valeur quantitative et qualitative) en fonction de la survie globale**

| **REPERTOIRE** | **HR** | **SD** | **P-value** |
|---|---|---|---|
| **DIVERSITE IGH** | **0,988** | **0,009** | **0,180** |
| **DIVPENIEIGH < 60%** | **1,540** | **0,323** | **0,180** |
| **DIVPENIEIGH ≥60%** | | | |

La diversité combinatoire de la chaîne IgH a, malgré les résultats de l'analyse univariée, été intégrée dans un modèle de prédiction multivarié simple (tableau 6) comme cela avait été réalisé pour l'étude du TCR (exemple 1). Dans cette analyse, le seuil de diversité a été placé à 50% afin de se placer dans les conditions les plus favorables. Avec une p-value >5% (P=0.2) la diversité combinatoire IgH ne permet pas de prédire le décès précoce chez les patientes métastatiques.

**Tableau 8: analyse multivariée de la diversité combinatoire IgH (seuil 50%) en fonction de la survie globale**

| **FACTEUR** | **groupes** | **Taux de survie à 14 mois** | **HR** | **SD** | **P** | **IC inf 95%** | **IC sup 95%** |
|---|---|---|---|---|---|---|---|
| **hémogiabine_gp** | **< 11,5** | **28%** | **3,609** | **0,363** | **4.10⁻⁴** | **1,772** | **7,349** |
| | **>= 11,5** | **69%** | | | | | |
| **PNN_gp** | **<= 7,5** | **59%** | **1,182** | **0,544** | **0,760** | **0,407** | **3,431** |
| | **> 7,5** | **43%** | | | | | |
| **site,tumoral Foie** | **Non** | **83%** | **0,239** | **0,482** | **0,003** | **0,093** | **0,616** |
| | **Oui** | **49%** | | | | | |
| **DIVPENIE_IGH** | **< 50%** | **38%** | **1,849** | **0,478** | **0,200** | **0,725** | **4,717** |
| | **>= 50%** | **59%** | | | | | |

### Conclusion

Ces observations sur la diversité IgH sont valables sur la cohorte étudiée, dans le modèle utilisé, et avec la version de la technologie à la date de l'étude. Dans ces conditions, la diversité IgH ne permet pas de prédire la survie des patientes atteintes de cancer du sein métastatique.

### Exemple 3 : Comparaison des coûts estimatifs pour un essai clinique pour tester un traitement innovant pour un cancer solide, avec ou sans sélection des patients selon leur niveau de diversité lymphocytaire

En considérant que le procédé de stratification à l'inclusion est moins cher que le traitement, si sur 100 patients testés par le présent procédé, 15 sont à risque, il sera possible de concentrer l'étude clinique sur les 15 patients plutôt que sur les 100 patients. Cette approche de stratification des patients ayant le plus besoin du traitement permet aussi d'augmenter les chances de succès d'obtenir une p-value significative vis-à-vis d'une approche globale qui au final pourrait aboutir à une p-value non significative. (Cf. tableau 7 ci-dessous).

**Tableau 9 : exemple d'hypothèses d'économie réalisée et d'amélioration de la p-value, suite à la stratification à l'inclusion des patients en fonction de la divpénie mesurée**

| **Exemple : 100 patients** | **Nombre** | **Cout** Exemple de cout moyen 10000€/patient | **Corrélation Avec efficacité de traitement** |
|---|---|---|---|
| **Divpénie <20%** | 15 (hypothèse) | 150 K€ | p< 0,005 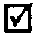 (hypothèse) |
| **Divpénie >20%** | 85 (hypothèse) | 850 K€ | p> 0,4 (hypothèse) |
| **Total** | **100** | **1 M€** | p> 0,3 (hypothèse) |

### Exemple 4 : Analyse de la « Numération Diversité Lymphocytaire (NDL) »

La demande WO 2009/095567 décrit (exemple 9) une nouvelle technique de numération, dite « Numération Diversité Lymphocytaire », couplant l'analyse du répertoire immunitaire à la numération du nombre de lymphocytes du patient. Les résultats d'une telle numération peuvent être représentés dans un graphique qui permet de visualiser plusieurs « zones » correspondant aux situations suivantes :
Zone 1. Numération faible (<1000 Ly/µL) et diversité combinatoire faible.
Zone 2. Numération faible (<1000 Ly/µL) mais diversité combinatoire V-J normale.
Zone 3. Numération normale (1000-3200Ly/µL) et diversité combinatoire faible.
Zone 4. Numération normale (1000-3200Ly/µL) et diversité normale.

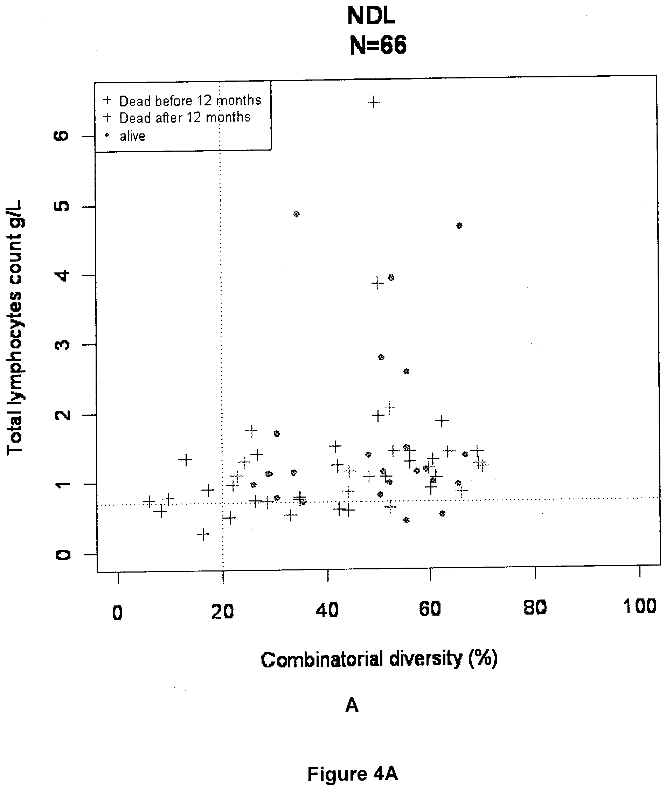

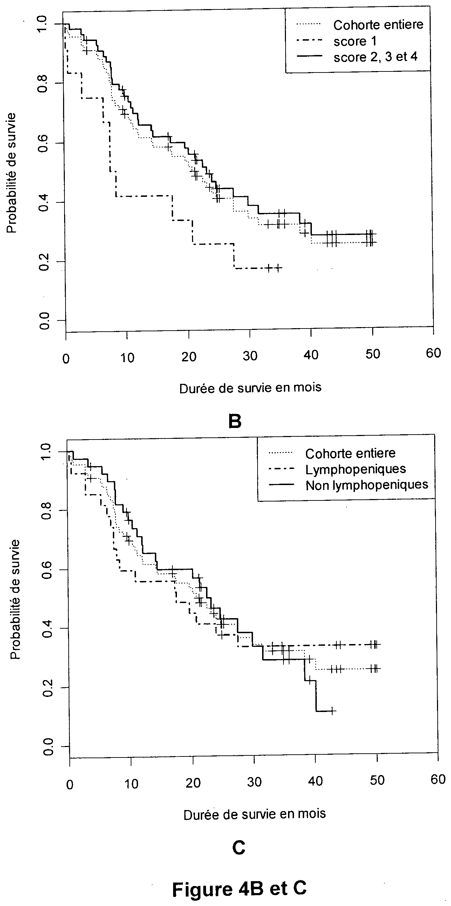

La figure 4A présente, pour la cohorte de 66 patientes étudiée aux exemples 1 et 2 ci-dessus (notée WP1a), un graphique NDL, dans lequel le seuil en-dessous duquel la diversité combinatoire est considérée comme faible a été fixé à 30%. La figure 4B présente, pour cette même cohorte, une courbe de survie des patientes en fonction de leur score NDL (correspondant à la zone dans laquelle se trouve leur résultat). La figure 4C présente, toujours pour la même cohorte, les courbes de survie des patientes en fonction de leur état lymphopénique ou non (avec un seuil à 0,7 Giga/1).

La cohorte du WP1b est composée de n=32 patientes atteintes de cancer du sein en première rechute métastatique avant tout traitement de chimiothérapie ; le prélèvement et les analyses immunologiques (phénotype et répertoire) ont été réalisés avant le traitement de chimiothérapie.

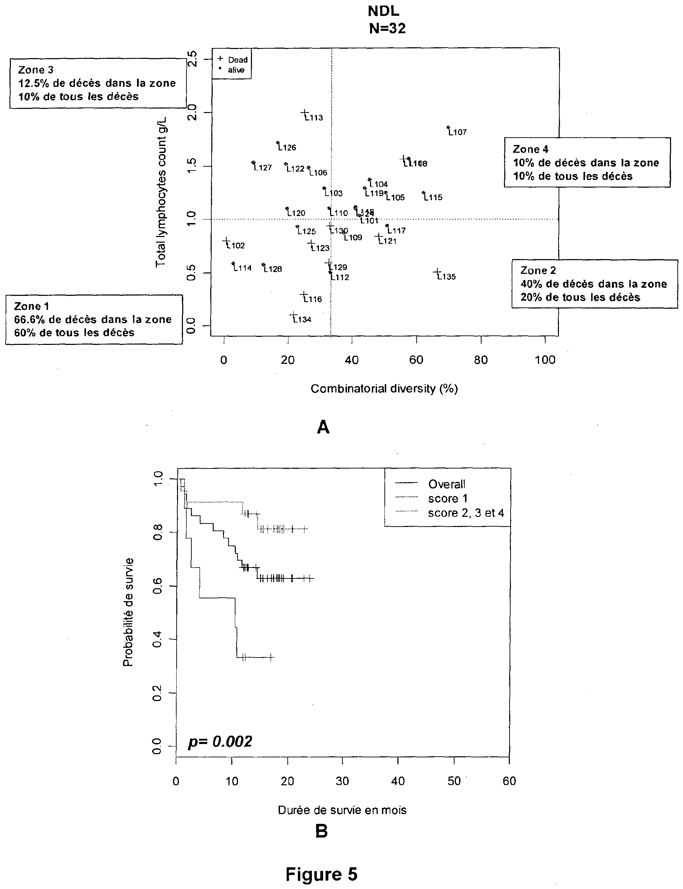

Le graphique NDL pour cette cohorte est présenté en figure 5A, et la courbe de survie des patientes en fonction de leur score NDL est présentée en figure 5B.

Les graphiques de NDL issus des analyses des cohortes WP1a (n=66, cohorte présentée dans les exemples 1 et 2) et WP1b (n=32) montrent que contre toute attente, et contrairement à ce qui est généralement considéré comme évident, la numération cellulaire n'est pas corrélée avec la diversité. Dans les graphiques de NDL des figures 4A et 5A, on peut observer des patientes avec une faible diversité et une numération normale (zone NDL3) et inversement des patientes avec une forte diversité et une lymphopénie (Zone NDL2). Ceci démontre clairement que la numération n'est pas systématiquement corrélée avec la diversité et la qualité du répertoire immunitaire.

***N.b*.:** Il est important de préciser que compte tenu du fait que la diversité immunitaire peut varier en fonction d'un traitement, ou d'une pathologie, la valeur pronostique du marqueur de divpénie n'est précise que pendant une certaine période de quelques jours à quelques mois (notion de durée de péremption de la prévision de risque infectieux). Il est donc nécessaire de réaliser une 2^{e} mesure de divpénie ou de lymphodivpénie en cas de changement de traitement ou d'évolution pathologique, ou après une certaine période. En effet, contrairement à des marqueurs génétique invariants, héréditaires ou non, tels que Her2/Neu, mutation p53, délétion d'un bras chromosomique, de pronostique de risque d'apparition d'une pathologie et/ou d'efficacité d'un traitement, la diversité combinatoire immunitaire peut évoluer.

Dans la cohorte rétrospective WP1a (n=66), avec un recul d'environ 50 mois, on peut faire les observations suivantes (figure 4) :
- Au seuil de diversité combinatoire hTRBV-J = 33% , l'analyse combinée des deux paramètres (diversité et lymphopénie) permet de caractériser les patientes les plus à risque dans la zone NDL1 (70% de décès dans la zone NDL1 et 33% de tous les décès).
- A numération comparable, la zone NDL2 est moins à risque que le score NDL1 (lymphopénique) avec seulement 25% de décès dans la zone NDL2 et 19 % de tous les décès de la cohorte WP1a à 12 mois.
- Inversement le score NDL4 semble être plus « protecteur » avec seulement 21% des décès dans la zone et 33% de tous les décès de la cohorte.
- A numération comparable, la zone de score NDL 3 est plus à risque que la zone 4 avec 40% de décès dans cette zone et 10% de tous les décès.

La « lymphodivpénie », c'est-à-dire une combinaison de faible diversité des lymphocytes et de lymphopénie, est donc un marqueur bien plus puissant que chacun des deux marqueurs considéré isolément. Il est en effet important de noter que dans cette cohorte, la lymphopénie aux seuils de 0,7 Giga/L et 1 Giga/L n'apparaît pas comme facteur pronostique de la survie globale, contrairement à ce qui a été démontré dans Ray-Coquard *et al.* (2009) (Voir Figure 4C).

Dans la 2^{ème} cohorte prospective WP1b (n=32), avec un recul minimum de 2 semaines et maximum de 24 mois, les observations suivantes sont faites :
- 66% des patientes lymphodivpéniques (zone du score NDL1) sont décédées précocément, ce qui représente 60% de tous les décès de la cohorte. La définition de la zone du score NDL1 correspond à une lymphopénie <1 Giga/L et une diversité combinatoire hTRBV-J inférieure ou égale à 33% mesurée après soustraction du bruit de fond inférieur ou égal à 0,05 UA.
- A numération comparable, la zone NDL2 est moins à risque que la zone NDL1 (40% de décès dans la zone NDL2 et 20% de tous les décès).
- Inversement le score NDL4 semble être plus « protecteur » avec seulement 10% des décès dans la zone et 10% de tous les décès de la cohorte.
- A numération comparable, la zone de score NDL 3 est légèrement plus à risque que la zone 4 avec 12.5% de décès dans cette zone, qui constituent 10% de tous les décès.

Les zones NDL 1 et 2 représentent un taux faible de lymphocytes, or un taux de lymphocytes inférieur à 1Giga/L est de mauvais pronostique (p = 0.0048). La zone NDL 1 qui apporte l'information de diversité présente quant à elle une meilleur p-value (p = 0.002).

### Exemple 5: Utilisation d'une autre méthode pour effectuer l'analyse de la « Numération Diversité Lymphocytaire (NDL) »

Afin de montrer que la méthode décrite ci-dessus peut être mise en œuvre en utilisant une technologie différente des PCR multi-N-plexes pour déterminer la diversité lymphocytaire des patients, les inventeurs ont utilisé la méthode de séquençage à haut débit décrite par Robins *et al.* (Blood, 2009) pour mesurer la diversité moléculaire de plusieurs échantillons.

En particulier, le séquençage a été réalisé sur un échantillon de PBMC issus d'un sujet sain et sur un échantillon de PBMC dans lequel un clone a été dilué (ce qui mime un échantillon issu d'un sujet atteint de leucémie T) ; les diversités combinatoires des deux échantillons ont ensuite été comparées avec celles obtenues par la technologie multi-N-plexe décrite plus haut.

Le séquençage à haut débit (NGS) a été effectué selon le procédé suivant :
- Constitution d'une banque d'ADN
- Obtention des données de séquences réalisées sur la banque d'ADN
- Analyse des données obtenues par séquençage.

Dans le cadre de l'analyse du répertoire immunitaire, la constitution de la banque d'ADN est une étape clef. Cette étape consiste en la réalisation des PCR multiplexes sur de l'ADN extrait de PBMC. Les produits de PCR ainsi obtenus doivent être représentatifs de la diversité du répertoire. Afin de minimiser la quantité nécessaire d'ADN utilisé lors de ces expériences, les PCR doivent être réalisées dans un nombre minimum de tubes. Au cours de ces PCR, les adaptateurs nécessaires à l'étape de séquençage sont intégrés.

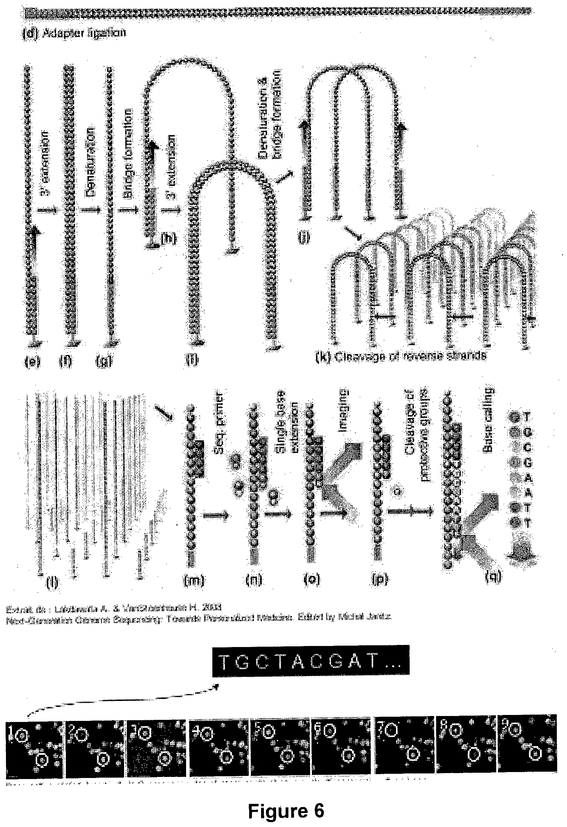

Le séquençage proprement dit a été réalisé selon la technique commercialisé par la société Illumina (voir figure 6). Le séquençage sur puce est fondé sur l'intégration de plusieurs techniques : les biopuces à ADN, la nanotechnologie, une variante de la technique de Sanger appelé CRT (*cyclic reversible termination*), ainsi que des technologies informatiques de pointe pour l'acquisition, le traitement et l'analyse des images. L'ADN (produit de PCR dans le cadre de l'analyse du répertoire immunitaire) est fixé en phase solide sur des cellules spécialement conçues pour assurer la fixation de chaque molécule d'ADN grâce aux adaptateurs. La formation des ponts d'amplifications permet d'avoir une haute densité de brins d'ADN. Le principe séquençage est basé sur l'incorporation réversible de nucléotides fluorescents et par lecture optique de la fluorescence. Comme pour la technique de Sanger, il s'agit d'une terminaison de synthèse basée sur l'utilisation d'un terminateur réversible contenant un groupement de protection attaché au nucléotide qui termine la synthèse d'ADN. L'élimination du groupement de protection par photoclivage utilisant la lumière ultraviolette (> 300nm) permet la restauration du groupement fonctionnel du nucléotide incorporé, ce qui permet à l'ADN polymérase d'incorporer le nucléotide suivant et ainsi de suite. Il s'agit là d'un séquençage en temps réel, basé sur la détection de la fluorescence mais en présence des 4 nucléotides marqués. La très haute densité de la puce (plus de 100 millions de molécules par centimètres carrés) permet de séquencer environ 100.000 paires de bases par seconde. Des molécules de 54 paires de bases sont séquencées puis alignées,

Les données de séquence obtenues sont analysées par un algorithme de *clustering* qui permet de regrouper entre elles des séquences appartenant :
- Au même gène V
- Au même gène J
- Au même CDR3
- A la même combinaison de gènes V-J
- A la même combinaison V-CDR3-J

Afin de pouvoir comparer les résultats obtenus par séquençage à ceux obtenus par la technologie multi-N-plexe, l'analyse des données a été réalisée en ne tenant compte que de la combinaison famille de gène V et gène J.

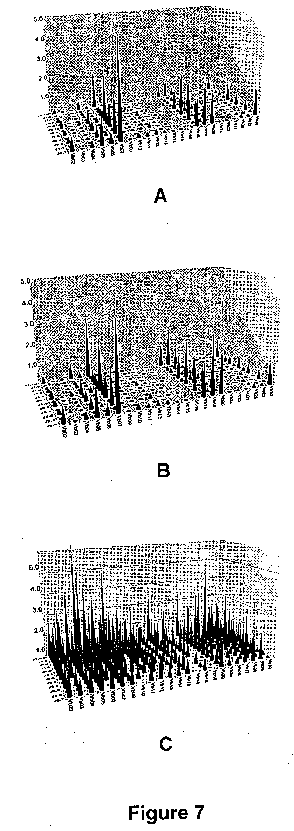

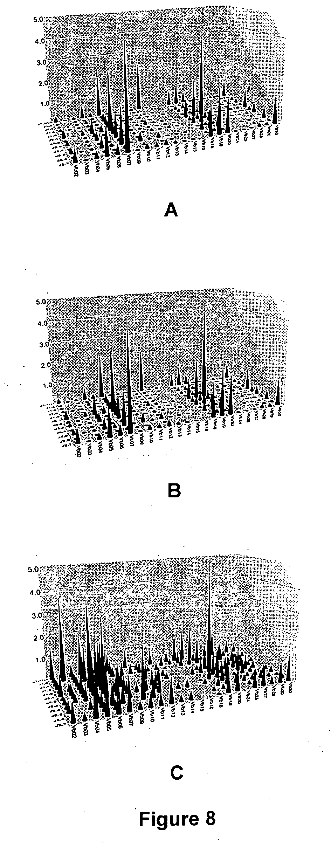

Les résultats obtenus sont présentés aux figures 7 et 8.

Les figures 7A et 7B correspondent à l'échantillon de PBMC de sujet sain, traité par séquençage en duplicat, ce qui donne des diversités de 69,9% et 73,6%, tandis que la mesure par la technologie multi-N-plexe donne un résultat de 84,4% (figure 7C).

Les figures 8A et 8B correspondent à l'échantillon mimant des PBMC de sujet leucémique, traité par séquençage en duplicat, ce qui donne des diversités de 68,8% et 67,8%, tandis que la mesure par la technologie multi-N-plexe donne un résultat de 62,68% (figure 8C).

En reproduisant ces expériences sur un nombre plus important d'échantillons, l'homme du métier peut sans difficulté « calibrer » une technologie alternative (telle que le séquençage à haut débit illustré ici) pour mettre en œuvre l'invention sans utiliser les PCR multi-N-plexes.

### REFERENCES

Baum, P.D. and McCune, J.M. (2006) Direct measurement of T-cell receptor repertoire diversity with AmpliCot. Nat Methods, 3, 895-901.
Bogue, M., Gilfillan, S., Benoist, C. and Mathis, D. (1992) Regulation of N-region diversity in antigen receptors through thymocyte differentiation and thymus ontogeny. Proc Natl Acad Sci USA, 89, 11011-11015.
Bonarius, H.P., Baas, F., Remmerswaal, E.B., van Lier, R.A., ten Berge, I.J., Tak, P.P. and de Vries, N. (2006) Monitoring the T-cell receptor repertoire at single-clone resolution. PLoS ONE, 1, e55.
Borg, C., Ray-Coquard, I., Philip, I., Clapisson, G., Bendriss-Vermare, N., Menetrier-Caux, C., Sebban, C., Biron, P. and Blay, J.Y. (2004) CD4 lymphopenia as a risk factor for febrile neutropenia and early death after cytotoxic chemotherapy in adult patients with cancer. Cancer, 101, 2675-2680.
Chaudhuri, J., Tian, M., Khuong, C., Chua, K., Pinaud, E. and Alt, F.W. (2003) Transcription-targeted DNA deamination by the AID antibody diversification enzyme. Nature, 422, 726-730.
Cochet, M., Pannetier, C., Regnault, A., Darche, S., Leclerc, C. and Kourilsky, P. (1992) Molecular detection and in vivo analysis of the specific T cell response to a protein antigen. Eur J Immunol, 22, 2639-2647.
Cox, D.R. (1972) Regression model and life tables. J Roy Stat Soc, 34, 187-220.
Davis, M.M. and Bjorkman, P.J. (1988) T-cell antigen receptor genes and T-cell recognition. Nature, 334, 395-402.
Douek, D.C., McFarland, R.D., Keiser, P.H., Gage, E.A., Massey, J.M., Haynes, B.F., Polis, M.A., Haase, A.T., Feinberg, M.B., Sullivan, J.L., Jamieson, B.D., Zack, J.A., Picker, L.J. and Koup, R.A. (1998) Changes in thymic function with age and during the treatment of HIV infection. Nature, 396, 690-695.
Fugmann, S.D., Lee, A.I., Shockett, P.E., Villey, I.J. and Schatz, D.G. (2000) The RAG proteins and V(D)J recombination: complexes, ends, and transposition. Annu Rev Immunol, 18, 495-527.
Fuschiotti, P., Pasqual, N., Hierle, V., Borel, E., London, J., Marche, P.N. and Jouvin-Marche, E. (2007) Analysis of the TCR alpha-chain rearrangement profile in human T lymphocytes. Mol Immunol, 44, 3380-3388.
Hamblin, T.J., Davis, Z., Gardiner, A., Oscier, D.G. and Stevenson, F.K. (1999) Unmutated Ig V(H) genes are associated with a more aggressive form of chronic lymphocytic leukemia. Blood, 94, 1848-1854.
Kaplan, E.L. and Meier, P. (1958) Non parametric estimation from incomplete observations. J Am Stat Assoc, 53, 457-481.
Kotani, A., Okazaki, I.M., Muramatsu, M., Kinoshita, K., Begum, N.A., Nakajima, T., Saito, H. and Honjo, T. (2005) A target selection of somatic hypermutations is regulated similarly between T and B cells upon activation-induced cytidine deaminase expression. Proc Natl Acad Sci USA, 102, 4506-4511.
Marodon, G., Desjardins, D., Mercey, L., Baillou, C., Parent, P., Manuel, M., Caux, C., Bellier, B., Pasqual, N. and Klatzmann, D. (2009) High diversity of the immune repertoire in humanized NOD.SCID.gamma c-/- mice. Eur J Immunol, 39, 2136-2145.
Pannetier, C., Even, J. and Kourilsky, P. (1995) T-cell repertoire diversity and clonal expansions in normal and clinical samples. Immunol Today, 16, 176-181.
Pasqual, N., Gallagher, M., Aude-Garcia, C., Loiodice, M., Thuderoz, F., Demongeot, J., Ceredig, R., Marche, P.N. and Jouvin-Marche, E. (2002) Quantitative and qualitative changes in V-J alpha rearrangements during mouse thymocytes differentiation: implication for a limited T cell receptor alpha chain repertoire. J Exp Med, 196, 1163-1173.
Pham, T., Belzer, M., Church, J.A., Kitchen, C., Wilson, C.M., Douglas, S.D., Geng, Y., Silva, M., Mitchell, R.M. and Krogstad, P. (2003) Assessment of thymic activity in human immunodeficiency virus-negative and -positive adolescents by real-time PCR quantitation of T-cell receptor rearrangement excision circles. Clin Diagn Lab Immunol, 10, 323-328.
Ray-Coquard, I., Cropet, C., Van Glabbeke, M., Sebban, C., Le Cesne, A., Judson, I., Tredan, O., Verweij, J., Biron, P., Labidi, I., Guastalla, J.P., Bachelot, T., Perol, D., Chabaud, S., Hogendoorn, P.C., Cassier, P., Dufresne, A. and Blay, J.Y. (2009) Lymphopenia as a prognostic factor for overall survival in advanced carcinomas, sarcomas, and lymphomas. Cancer Res, 69, 5383-5391.
Robins, H.S., Campregher, P.V., Srivastava, S.K., Wacher, A., Turtle, C.J., Kahsai, O., Riddell, S.R., Warren, E.H. and Carlson, C.S. (2009) Comprehensive assessment of T-cell receptor beta-chain diversity in alphabeta T cells. Blood, 114, 4099-4107.
Rothman, K.J. (1978) Estimation of confidence limits for the cumulative probability of survival in life table analysis. J Chronic Dis, 31, 557-560.
Therneau, T.M. and Grambsch, P.M. (2000) Modeling, survival data. extending the Cox Model. Statistics for biology and health, Springer Ed. Mayo Foundation, New York.
Van den Beemd, van Dongen et al. (2000), "Flow cytometric detection of clonality in mature T-cell malignancies by use of a Vb antibody kit", ISAC Abstract.
Wei, L., Wang-Jun, L., Li, M., Yong-Ta, H., Min, S., Qian, W., Xiao-Ning, W. (2010) Dynamic monitoring the TCR CDR3 spectratypes in patients with metastatic CRC treated with a combination of bevacizumab, irinotecan, fluorouracil, and leucovorin. Cancer Immunol, Immnunother, 59, 247-256

## Revendications

1. Méthode pour établir *ex vivo* ou *in vitro* un pronostic pour un individu souffrant d'un cancer solide métastatique, comprenant les étapes suivantes :
(i) à partir d'acide nucléique provenant d'un échantillon biologique contenant des lymphocytes dudit individu, prélevé avant l'administration de la première ligne de chimiothérapie, mesurer le niveau de diversité du répertoire des lymphocytes T dudit individu ;
(ii) comparer le niveau de diversité mesuré à l'étape (i) à un seuil prédéterminé ;
(iii) en déduire, dans le cas où le niveau de diversité mesuré à l'étape (i) est inférieur au seuil prédéterminé, que l'individu a un risque élevé de décès précoce.

2. Méthode selon la revendication 1, dans laquelle le cancer dont souffre l'individu est un cancer du sein.

3. Méthode selon la revendication 1 ou la revendication 2, dans laquelle, à l'étape (i), l'acide nucléique utilisé est de l'ADN génomique.

4. Méthode selon l'une quelconque des revendications 1 à 3, dans laquelle, à l'étape (i), la diversité combinatoire du répertoire des lymphocytes T est mesurée.

5. Méthode selon la revendication 4, dans laquelle la mesure de la diversité combinatoire du répertoire des lymphocytes T dudit individu est effectuée par une méthode permettant d'analyser au moins 10 réarrangements V(D)J du locus TRA ou du locus TRB.

6. Méthode selon la revendication 5, dans laquelle la mesure de la diversité combinatoire du répertoire des lymphocytes T dudit individu est effectuée par une méthode permettant d'analyser au moins 20 réarrangements V(D)J du locus TRA ou du locus TRB.

7. Méthode selon la revendication 5 ou la revendication 6, dans laquelle la mesure de la diversité combinatoire du répertoire des lymphocytes T dudit individu est effectuée par une méthode permettant d'analyser au moins 70% des réarrangements V(D)J du locus TRB.

8. Méthode selon l'une quelconque des revendications 5 à 7, dans laquelle la mesure de la diversité combinatoire du répertoire des lymphocytes T est effectuée par des PCR multi-n-plexes avec n≥2 à l'aide de combinaisons d'au moins 3 amorces, chaque combinaison d'amorces comprenant au moins les amorces hTRBJ1.6 (SEQ ID No : 1), hTRBJ2.7 (SEQ ID No : 2) et une amorce hTRBV choisie dans le groupe constitué des amorces de SEQ ID Nos : 3 à 25.

9. Méthode selon la revendication 8, dans laquelle l'analyse est réalisée en effectuant au moins 23 PCR multi-2-plexes, chaque PCR multi-2-plexe étant réalisée avec un triplet d'amorces constitué des amorces hTRBJ1.6 (SEQ ID No : 1), hTRBJ2.7 (SEQ ID No : 2) et d'une amorce hTRBV choisie dans le groupe constitué des amorces de SEQ ID Nos : 3 à 25.

10. Méthode selon l'une quelconque des revendications 1 à 9, dans laquelle l'échantillon biologique est un échantillon d'un tissu choisi parmi le sang, les globules blancs (PBMC), le thymus, un ganglion, la rate, le sein, le foie, la peau, un échantillon tumoral ou un fluide biologique (épanchement pleural, ascite).

11. Méthode selon l'une quelconque des revendications 1 à 10, dans laquelle l'échantillon biologique est un échantillon de sang total ou de PBMC.

12. Méthode selon l'une quelconque des revendications 1 à 11, dans laquelle à l'étape (i), l'ADN génomique est purifié à partir de l'échantillon biologique.

13. Méthode selon l'une quelconque des revendications 1 à 12, dans laquelle le seuil utilisé à l'étape (ii) est tel que l'espérance de survie d'un patient dont le niveau de diversité mesuré à l'étape (i) est inférieur à ce seuil est au moins deux fois moindre que l'espérance de survie généralement observée pour les patients atteints du même cancer solide métastatique que celui dont il est atteint.

14. Méthode selon l'une quelconque des revendications 1 à 13, dans laquelle à l'étape (ii), le seuil est fixé à 30% de diversité, et l'interprétation à l'étape (iii) consiste à dire que si le niveau de diversité combinatoire des lymphocytes T mesuré est inférieur audit seuil, l'individu a une espérance de vie deux fois moindre que celle généralement observée pour sa pathologie.

15. Méthode selon l'une quelconque des revendications 1 à 13, dans laquelle à l'étape (ii), le seuil est fixé à 20% de diversité, et l'interprétation à l'étape (iii) consiste à dire que si le niveau de diversité combinatoire des lymphocytes T mesuré est inférieur audit seuil, l'individu a une espérance de vie de deux à cinq fois moindre que celle généralement observée pour sa pathologie.

16. Méthode selon l'une quelconque des revendications 1 à 15, dans laquelle le patient est atteint d'un cancer du sein métastatique, et dans laquelle un niveau de diversité des réarrangements V(D)J du locus TRB inférieur à 20% est indicatif d'une espérance de survie du patient inférieure à 6 mois.

17. Méthode selon l'une quelconque des revendications 1 à 16, dans laquelle le pronostic est établi en combinant le niveau de diversité du répertoire des lymphocytes T dudit individu avec sa numération lymphocytaire.

18. Méthode selon l'une quelconque des revendications 1 à 17, dans laquelle le pronostic est établi en associant au niveau de diversité mesuré à l'étape (i), un ou plusieurs autre(s) paramètre(s) biologique(s) ou clinique(s) sélectionnés parmi la numération lymphocytaire, le nombre de cellules CD4+, le niveau cytokines sériques, le PS (*performance status*) et le niveau d'hémoglobine.

19. Méthode pour déterminer *ex vivo* ou *in vitro* si un patient atteint d'un cancer solide doit être inclus dans un protocole de recherche clinique, comprenant les étapes suivantes :
(i) déterminer, en mettant en œuvre la méthode selon l'une quelconque des revendications précédentes, si le patient a un risque accru de décès précoce, et
(ii) si le patient a un risque accru de décès précoce, l'inclure dans le protocole de recherche clinique.

20. Utilisation d'une méthode selon l'une quelconque des revendications 1 à 19, pour aider le clinicien à déterminer si un patient atteint d'un cancer solide doit bénéficier d'un suivi hospitalier particulier et/ou d'une antibiothérapie préventive et/ou d'une immunostimulation et/ou d'une vaccination et/ou d'une chimiothérapie et/ou d'un changement de posologie ou de fréquence d'administration d'une chimiothérapie, et/ou de compléments alimentaires visant à reconstituer ses défenses immunitaires.

## Patentansprüche

1. Verfahren zum *ex vivo* oder *in vitro* Festlegen einer Prognose für ein Individuum, das an einem metastasierten soliden Krebs leidet, umfassend die folgenden Schritte:
(i) ausgehend von einer Nukleinsäure, die aus einer biologischen Probe stammt, die Lymphozyten von dem Individuum enthält, entnommen vor der Verabreichung der ersten Reihe einer Chemotherapie, Messen des Diversitätsniveaus des T-Lymphozyten-Repertoires des Individuums;
(ii) Vergleichen des in Schritt (i) gemessenen Diversitätsniveaus mit einem vorbestimmten Schwellenwert;
(iii) Ableiten, falls das in Schritt (i) gemessene Diversitätsniveau unter dem vorbestimmten Schwellenwert liegt, dass das Individuum ein hohes Risiko für einen frühen Tod hat.

2. Verfahren nach Anspruch 1, wobei der Krebs, an dem das Individuum leidet, Brustkrebs ist.

3. Verfahren nach Anspruch 1 oder Anspruch 2, wobei in Schritt (i) die verwendete Nukleinsäure genomische DNA ist.

4. Verfahren nach einem der Ansprüche 1 bis 3, wobei in Schritt (i) die kombinatorische Diversität des T-Lymphozyten-Repertoires gemessen wird.

5. Verfahren nach Anspruch 4, wobei die Messung der kombinatorischen Diversität des T-Lymphozyten-Repertoires des Individuums durch ein Verfahren, das ein Analysieren von mindestens 10 V(D)J-Umlagerungen des TRA-Locus oder des TRB-Locus erlaubt, durchgeführt wird.

6. Verfahren nach Anspruch 5, wobei die Messung der kombinatorischen Diversität des T-Lymphozyten-Repertoires des Individuums durch ein Verfahren, das ein Analysieren von mindestens 20 V(D)J-Umlagerungen des TRA-Locus oder des TRB-Locus erlaubt, durchgeführt wird.

7. Verfahren nach Anspruch 5 oder Anspruch 6, wobei die Messung der kombinatorischen Diversität des T-Lymphozyten-Repertoires des Individuums durch ein Verfahren, das es ermöglicht, mindestens 70% der V(D)J-Umlagerungen des TRB-Locus zu analysieren, durchgeführt wird.

8. Verfahren nach einem der Ansprüche 5 bis 7, wobei die Messung der kombinatorischen Diversität des T-Lymphozyten-Repertoires durch Multi-n-Plex-PCRs mit n≥2 mit Hilfe von Kombinationen von mindestens 3 Primern durchgeführt wird, wobei jede Primerkombination mindestens die Primer hTRBJ1.6 (SEQ ID No: 1), hTRBJ2.7 (SEQ ID No: 2) und einen hTRBV-Primer, ausgewählt aus der Gruppe bestehend aus den Primern der SEQ ID Nos: 3 bis 25, umfasst.

9. Verfahren nach Anspruch 8, wobei die Analyse durchgeführt wird, indem mindestens 23 Multi-2-Plex-PCRs durchgeführt werden, wobei jede Multi-2-Plex-PCR mit einem Triplett von Primern durchgeführt wird, bestehend aus den Primern hTRBJ1.6 (SEQ ID No: 1), hTRBJ2.7 (SEQ ID No: 2) und einem hTRBV-Primer, ausgewählt aus der Gruppe bestehend aus den Primern der SEQ ID Nos: 3 bis 25.

10. Verfahren nach einem der Ansprüche 1 bis 9, wobei die biologische Probe eine Probe eines Gewebes ist, ausgewählt aus dem Blut, den weißen Blutkörperchen (PBMC), dem Thymus, einem Ganglion, der Milz, der Brust, der Leber, der Haut, einer Tumorprobe oder einer biologischen Flüssigkeit (Pleuraerguss, Aszites).

11. Verfahren nach einem der Ansprüche 1 bis 10, wobei die biologische Probe eine Vollblut- oder PBMC-Probe ist.

12. Verfahren nach einem der Ansprüche 1 bis 11, wobei in Schritt (i) die genomische DNA aus der biologischen Probe gereinigt ist.

13. Verfahren nach einem der Ansprüche 1 bis 12, wobei der in Schritt (ii) verwendete Schwellenwert so ist, dass die Überlebenserwartung eines Patienten, dessen in Schritt (i) gemessenes Diversitätsniveau unterhalb dieser Schwelle liegt, mindestens zweimal weniger als die Überlebenserwartung ist, die im Allgemeinen bei Patienten mit demselben metastasierten soliden Krebs wie demjenigen, von dem er betroffen ist, beobachtet wird.

14. Verfahren nach einem der Ansprüche 1 bis 13, wobei in Schritt (ii) der Schwellenwert auf 30% Diversität eingestellt ist und die Interpretation in Schritt (iii) daraus besteht zu sagen, dass, wenn das kombinatorische Diversitätsniveau der gemessenen T-Lymphozyten unter dieser Schwelle liegt, das Individuum eine Lebenserwartung hat, die doppelt so niedrig ist wie die, die allgemein für seine Pathologie beobachtet wird.

15. Verfahren nach einem der Ansprüche 1 bis 13, wobei in Schritt (ii) der Schwellenwert auf 20% Diversität eingestellt ist und die Interpretation in Schritt (iii) daraus besteht zu sagen, dass, wenn das kombinatorische Diversitätsniveau der gemessenen T-Lymphozyten unter dieser Schwelle liegt, das Individuum eine Lebenserwartung hat, die doppelt bis fünffach so niedrig ist wie die, die allgemein für seine Pathologie beobachtet wird.

16. Verfahren nach einem der Ansprüche 1 bis 15, wobei der Patient metastasierten Brustkrebs hat und wobei ein Diversitätsniveau der V(D)J-Umlagerungen des TRB-Locus von weniger als 20% eine Überlebenserwartung des Patienten von weniger als 6 Monaten anzeigt.

17. Verfahren nach einem der Ansprüche 1 bis 16, wobei die Prognose durch Kombinieren des Diversitätsniveaus des T-Zell-Repertoires des Individuums mit seiner Lymphozytenzahl festgelegt wird.

18. Verfahren nach einem der Ansprüche 1 bis 17, wobei die Prognose durch Assoziieren, eines anderen biologischen oder klinischen Parameters oder mehrerer anderer biologischer oder klinischer Parameter ausgewählt aus der Lymphozytenzahl, der CD4+-Zellzahl, dem Serumzytokinniveau, dem PS (Leistungsstatus) und dem Hämoglobinniveau mit dem in Schritt (i) gemessenen Diversitätsniveau festgelegt wird.

19. Verfahren zur *ex vivo* oder *in vitro* Bestimmung, ob ein Patient mit einem soliden Krebs in ein klinisches Forschungsprotokoll aufgenommen werden sollte, das die folgenden Schritte umfasst:
(i) Bestimmen unter Umsetzung des Verfahrens gemäß einem der vorhergehenden Ansprüche, ob der Patient ein erhöhtes Risiko für einen frühen Tod aufweist, und
(ii) wenn der Patient ein erhöhtes Risiko für einen frühen Tod aufweist, ihn in das klinische Forschungsprotokoll aufnehmen.

20. Verwendung eines Verfahrens nach einem der Ansprüche 1 bis 19, um den Kliniker bei einer Bestimmung zu unterstützen, ob ein Patient mit solidem Krebs von einer speziellen Krankenhausüberwachung und/oder einer vorbeugenden Antibiotikatherapie und/oder einer Immunstimulation und/oder einer Impfung und/oder einer Chemotherapie und/oder einer Änderung der Dosierung oder Häufigkeit einer Verabreichung einer Chemotherapie und/oder Nahrungsergänzungsmitteln zur Wiederherstellung der Immunabwehr profitieren sollte.

## Claims

1. A method for establishing *ex vivo* or *in vitro* a prognosis for an individual who has a metastatic solid cancer, comprising the following steps:
(i) from nucleic acid derived from a biological sample, taken before administration of the first line of chemotherapy, containing lymphocytes of said individual, measuring the level of diversity of the T-lymphocyte repertoire of said individual;
(ii) comparing the level of diversity measured in step (i) with a predetermined threshold;
(iii) deducing from this, if the level of diversity measured in step (i) is below the predetermined threshold, that the individual has a high risk of early death.

2. A method according to claim 1, in which the cancer from which the individual is suffering is a breast cancer.

3. A method according to claim 1 or claim 2, in which, in step (i), the nucleic acid used is genomic DNA.

4. A method according to any one of claims 1 to 3, in which, in step (i), the combinatorial diversity of the T-lymphocyte repertoire is measured.

5. A method according to claim 4, in which measurement of the combinatorial diversity of the T-lymphocyte repertoire of said individual is carried out by a method making it possible to analyse at least 10 V(D)J rearrangements of the TRA locus or of the TRB locus.

6. A method according to claim 5, in which measurement of the combinatorial diversity of the T-lymphocyte repertoire of said individual is carried out by a method making it possible to analyse at least 20 V(D)J rearrangements of the TRA locus or of the TRB locus.

7. A method according to claim 5 or claim 6, in which measurement of the combinatorial diversity of the T-lymphocyte repertoire of said individual is carried out by a method making it possible to analyse at least 70% of the V(D)J rearrangements of the TRB locus.

8. A method according to any one of claims 5 to 7, in which measurement of the combinatorial diversity of the T-lymphocyte repertoire is carried out by multi-n-plex PCRs with n≥2 by means of combinations of at least 3 primers, each combination of primers comprising at least the primers hTRBJ1.6 (SEQ ID No. 1), hTRBJ2.7 (SEQ ID No. 2) and a primer hTRBV selected from the group consisting of the primers of SEQ ID Nos.: 3 to 25.

9. A method according to claim 8, in which the analysis is performed by carrying out at least 23 multi-2-plex PCRs, each multi-2-plex PCR being carried out with a triplet of primers consisting of the primers hTRBJ1.6 (SEQ ID No. 1), hTRB J2.7 (SEQ ID No. 2) and a primer hTRBV selected from the group consisting of the primers of SEQ ID Nos.: 3 to 25.

10. A method according to any one of claims 1 to 9, in which the biological sample is a sample of a tissue selected from blood, white blood cells (PBMC), thymus, a lymph node, spleen, breast, liver, skin, a tumour sample or a biological fluid (pleural effusion, ascites).

11. A method according to any one of claims 1 to 10, in which the biological sample is a sample of whole blood or of PBMC.

12. A method according to any one of claims 1 to 11, in which, in step (i), the genomic DNA is purified from the biological sample.

13. A method according to any one of claims 1 to 12, in which the threshold used in step (ii) is such that the expected survival of a patient for whom the level of diversity measured in step (i) is below this threshold is at most half the expected survival generally observed for patients with the same metastatic solid cancer as that affecting the patient.

14. A method according to any one of claims 1 to 13, in which, in step (ii), the threshold is set at 30% diversity, and the interpretation in step (iii) consists of saying that if the measured level of T-lymphocyte combinatorial diversity is below said threshold, the individual has a life expectancy half that generally observed for his/her pathology.

15. A method according to any one of claims 1 to 13, in which, in step (ii), the threshold is set at 20% diversity, and the interpretation in step (iii) consists of saying that if the measured level of T-lymphocyte combinatorial diversity is below said threshold, the individual has a life expectancy of half to one fifth that generally observed for his/her pathology.

16. A method according to any one of claims 1 to 15, in which the patient has metastatic breast cancer, and in which a level of diversity of the V(D)J rearrangements of the TRB locus of less than 20% is indicative of an expected survival of the patient of less than 6 months.

17. A method according to any one of claims 1 to 16, in which the prognosis is established by combining the level of diversity of the T-lymphocyte repertoire of said individual with his/her lymphocyte count.

18. A method according to any one of claims 1 to 17, in which the prognosis is established by combining the level of diversity measured in step (i), with one or more other biological or clinical parameters selected from the lymphocyte count, CD4+ cell count, serum cytokine level, PS (performance status) and haemoglobin level.

19. A method for determining *ex vivo* or *in vitro* whether a patient with a solid cancer should be included in a clinical research protocol for testing a new medicinal product, comprising the following steps:
(i) determining, by employing the method according to any one of the preceding claims, whether the patient has an increased risk of early death, and
(ii) if the patient has an increased risk of early death, including him/her in the clinical research protocol.

20. Use of a method according to any one of claims 1 to 19, for assisting the clinician in determining whether a patient with a solid cancer should benefit from particular hospital follow-up and/or prophylactic antibiotic therapy and/or immunostimulation and/or a vaccination and/or chemotherapy and/or a change in dosage or frequency of administration of chemotherapy, and/or dietary supplements aiming to restore the immune defences.


### SEQUENCE LISTING

<110> IMMUNID
   MANUARII, Manuel
   CAUX, Christophe
   MOURET , Jean-François
   MENETRIER-CAUX, Christine
   PASQUAL , Nicolas
   BACHELOT, Thomas
   BLAY , Jean-Yves
<120> UTILISATION DE LA DIVERSITE COMBINATOIRE DU REPERTOIRE DES LYMPHOCYTES T COMME MARQUEUR PRONOSTIC D'UN CANCER
<130> VMAahF2110/2
<160> 25
<170> PatentIn version 3.4
<210> 1
   <211> 25
   <212> DNA
   <213> Artificial
<220>
   <223> amorce
<400> 1
   cttggtgcat ggctatgtaa tcctg 25
<210> 2
   <211> 22
   <212> DNA
   <213> Artificial
<220>
   <223> amorce
<400> 2
   ctcgccctct gctcagcttt cc 22
<210> 3
   <211> 26
   <212> DNA
   <213> Artificial
<220>
   <223> amorce
<400> 3
   cacacagatg ggacaggaag tgatct 26
<210> 4
   <211> 23
   <212> DNA
   <213> Artificial
<220>
   <223> amorce
<400> 4
   gcttctcacc tgaatgcccc aac 23
<210> 5
   <211> 25
   <212> DNA
   <213> Artificial
<220>
   <223> amorce
<400> 5
   ctgatcaaaa cgagaggaca gcaag 25
<210> 6
   <211> 25
   <212> DNA
   <213> Artificial
<220>
   <223> amorce
<400> 6
   ctgatcaaaa cgagaggaca gcacg 25
<210> 7
   <211> 23
   <212> DNA
   <213> Artificial
<220>
   <223> amorce
<400> 7
   gatcacccag gcaccaacat ctc 23
<210> 8
   <211> 25
   <212> DNA
   <213> Artificial
<220>
   <223> amorce
<400> 8
   cagatcacac aggagctgga gtctc 25
<210> 9
   <211> 27
   <212> DNA
   <213> Artificial
<220>
   <223> amorce
<400> 9
   cacagatcac gcagatactg gagtctc 27
<210> 10
   <211> 23
   <212> DNA
   <213> Artificial
<220>
   <223> amorce
<400> 10
   cgcacaacag ttccctgact tgc 23
<210> 11
   <211> 27
   <212> DNA
   <213> Artificial
<220>
   <223> amorce
<400> 11
   ttcacagttg cctaaggatc gattttc 27
<210> 12
   <211> 27
   <212> DNA
   <213> Artificial
<220>
   <223> amorce
<400> 12
   ttctctggta cagacagacc tttgtgc 27
<210> 13
   <211> 27
   <212> DNA
   <213> Artificial
<220>
   <223> amorce
<400> 13
   ttttctggta cagagatacc ttcgtgc 27
<210> 14
   <211> 25
   <212> DNA
   <213> Artificial
<220>
   <223> amorce
<400> 14
   gttgctgaag tgtcaaactc tcccg 25
<210> 15
   <211> 24
   <212> DNA
   <213> Artificial
<220>
   <223> amorce
<400> 15
   tccccagcca cagcgtaata gaga 24
<210> 16
   <211> 24
   <212> DNA
   <213> Artificial
<220>
   <223> amorce
<400> 16
   ccccaaagct gctgttccac tact 24
<210> 17
   <211> 22
   <212> DNA
   <213> Artificial
<220>
   <223> amorce
<400> 17
   ctcctggtga agaagtcgcc ca 22
<210> 18
   <211> 22
   <212> DNA
   <213> Artificial
<220>
   <223> amorce
<400> 18
   tagtgcgagg agattcggca gc 22
<210> 19
   <211> 23
   <212> DNA
   <213> Artificial
<220>
   <223> amorce
<400> 19
   ctgggagcaa gtgagtcctg ggt 23
<210> 20
   <211> 24
   <212> DNA
   <213> Artificial
<220>
   <223> amorce
<400> 20
   tcatcaacca tgcaagcctg acct 24
<210> 21
   <211> 24
   <212> DNA
   <213> Artificial
<220>
   <223> amorce
<400> 21
   agtgtctctc gacaggcaca ggct 24
<210> 22
   <211> 23
   <212> DNA
   <213> Artificial
<220>
   <223> amorce
<400> 22
   cctctttgtt gggtttgtgc ctg 23
<210> 23
   <211> 22
   <212> DNA
   <213> Artificial
<220>
   <223> amorce
<400> 23
   gtccccttcc tttacaggcc cc 22
<210> 24
   <211> 21
   <212> DNA
   <213> Artificial
<220>
   <223> amorce
<400> 24
   ccatcagccg cccaaaccta a 21
<210> 25
   <211> 26
   <212> DNA
   <213> Artificial
<220>
   <223> amorce
<400> 25
   tgctcttcta ctccgttggt attggc 26